# EUROPEAN PATENT APPLICATION

(11) **EP 1 374 767 A2**
(43) Date of publication of application: **02.01.2004**
(21) Application number: 02701692.2
(22) Date of filing: 04.03.2002
(51) Int. Cl.: A61B 5/08

(54) **RESPIRATION FUNCTION MEASURING SYSTEM AND ITS APPLICATION**

(30) Priority: 02.03.2001 JP 2001058708
(71) Applicant: Kihara, Norio, Yokohama-shi, Kanagawa 225-0002 (JP)
(72) Inventor: KIHARA, Norio, Yokohama-shi, Kanagawa 225-0002 (JP); MIYAGAWA, Tetsuo, Yokohama-shi, Kanagawa 225-0003 (JP); NAGAI, Atsushi, Shinjuku-ku, Tokyo 162-0054 (JP); MIYAGI, Seishirou, Ishikawa-shi, Okinawa 904-1107 (JP); KITAMURA, Satoshi, Kawachi-gun, Tochigi 329-0431 (JP)
(74) Representative: Pfenning, Meinig & Partner
(86) International application number: PCT/JP2002/001951
(87) International publication number: WO 2002/069878

(57) **Abstract**

The measuring system (10) measures a respiratory waveform by the costal respiratory measuring unit (100a) and the abdominal respiratory measuring unit (100b). Each measuring unit (100) includes the sensing unit (110) for detecting volume change of the measurement part, and the fixing unit (120) for arranging the sensing unit (110) to the measurement part. The sensing unit (110) detects volume change of the measurement part with the pressure change given by the fixing unit (120) and the measurement part. The control unit (200) controls measurement and receives output from the measuring unit (100). The respiratory waveform of chest respiration and abdominal respiration is independently acquirable from the above composition. The acquired respiratory waveform is analyzed in the analyzing unit (300), and is referred to the respiratory function database (350), and the medical view is acquired for the subject's respiratory function.

## Description

### TECHNICAL FIELD

The present invention relates to a technology for measuring respiratory function, and it particular relates to a measuring system, a measuring apparatus and a measuring method for measuring respiratory function of a subject, an analyzing apparatus, analyzing server, analyzing program for analyzing a measurement result, and a rehabilitation assisting apparatus and a rehabilitation assisting method which can use the measurement system. The measurement system of the present invention is applicable to diagnose respiratory disorders such as SAS (sleep apnea syndrome), ALS (amyotrophic lateral selerosis), respiratory tract blockage, and/or asthma, etc.

### BACKGROUND TECHNOLOGY

It is indispensable to recognize a patient's respiratory function accurately to diagnose a patient who suffers with respiratory disorders. Traditionally, the Spirometer has been widely used as the equipment to examine respiratory functions. And with the Spirometer, lung capacity fractions of a patient such as VC (vital capacity), ERV (expiratory reserve volume), IRV (inspiratory reserve volume), IC (inspiratory capacity), TV (tidal volume), FRC (functional residual capacity), RV (residual volume), TLC (total lung capacity), as well as forced expiration curve, FVC (forced vital capacity), FEV1.0 (forced expiratory volume), flow volume curve, and PEFR (peak expiratory flow rate) are measured and patient's respiratory functions are diagnosed.

However, since the Spirometer measures volume or rate of respiration and/or inspiration, data of costal respiration and abdominal respiration cannot be measured independently. A respisomnography is sometimes used to obtain the diagnosis characteristically for thoracic respiration and the abdominal respiration. But, when the respisomnography is used, the data are largely effected by the measurement conditions such as how two measuring belts are applied, etc. and the reproducibility of the data is poor, therefore different data are obtained every time even the same subject is measured. For that reason, though a certain diagnosis can be obtained by the respisomnography for thoracic respiration and abdominal respiration, it has been impossible to evaluate the measurement data quantitatively to produce useful medical views.

### DISCLOSURE OF THE INVENTION

Therefore, an objective of the present invention is to provide a technique that enables obtaining highly reliable respiratory waveform data of multiple measurement parts of a human body. Another object of the present invention is providing a technology that makes the rehabilitation of respiratory function more effective utilizing the above-mentioned measurement technology, and further object is providing a technology to analyze appropriately the data measured by above-mentioned measurement technology.

An embodiment according to the present invention relates to a measuring system. This measuring system is a measuring system for measuring respiratory function characterized in that it includes: a first measuring unit for detecting volume change of a first measurement part of a subject sequent to respiratory movement; a second measuring unit for detecting volume change of a second measurement part of the subject sequent to respiratory movement; a control unit which acquires an output from said first measuring unit and said second measuring unit; and an analyzing unit which analyzes the output which said control unit acquires; said first measuring unit and said second measuring unit respectively include: a sensing unit for sensing the volume change of the measurement part; and a fixing unit for arranging said sensing unit to the measurement part; wherein said fixing unit can fix said sensing unit in a manner of impressing said sensing unit on the measurement part.

By measuring volume change of the measurement part, a subject's expiration volume and inhalation volume can be measured. Moreover, since the volume change at the time of breathing of the first measurement part and the second measurement part can be measured independently, the state of thoracic respiration and abdominal respiration is quantitatively acquirable, respectively, for example. Thereby, the quantitative evaluation about thoracic respiration and abdominal respiration can be attained though it used to be impossible conventionally. And the advantage of this embodiment is tremendously meaningful in the medical field since it enables not only calculating indices of the rate of abdomen contribution with sufficient accuracy, but also an important finding may be acquired, for instance, from the difference in the respiratory waveform of chest and abdomen.

The first measurement part may be near thorax, and said first measuring unit detects the volume change near thorax sequent to costal respiration, and the second measurement part may be near abdomen, and said second measuring unit detects the volume change near abdomen sequent to abdominal respiration.

Said sensing unit may sense the volume change of the measurement part from change of pressure applied by said fixing unit and the measurement part. As the sensing unit is fixed so that it may press down to the measurement part by the fixing unit, the sensing unit may be pressed by the measurement part and the fixing unit when the volume of the measurement part increases. Therefore, the volume change of the measurement part can be read by change of the pressure given to the sensing unit.

Said sensing unit may have a bag-like form; said fixing unit may have a belt-like form; and said sensing unit may be impressed to the measurement part in such a manner that a body of the subject is wrapped around by said fixing unit. A harvesting unit which harvest the sensing unit may be comprised in the fixing unit, and the fixing unit may be wrapped with the body of the subject with the sensing unit harvested in the harvesting unit.

Said sensing unit may have a cavity inside; said measuring system may further include a pressure sensor for measuring air pressure in the cavity; and the volume change of the measurement part may be detected from change of the air pressure in the cavity. The measuring system may further include a pump for sending gas into the cavity. The measuring system may further include an initial pressure adjusting unit for adjusting the air pressure in the cavity to predetermined initial pressure by sending gas into the cavity before a measurement of respiratory function. In order to measure volume change of the measurement part at the respiration time correctly, it is desirable to adjust into the state where a certain amount of pressure is impressed to the sensing unit also at the time of the maximum expiration. Therefore, gas is sent inside the sensing unit before measurement of the respiratory function so that the predetermined initial pressure is set.

Said initial pressure adjusting unit may adjust the initial pressure within a range where a ratio of the volume change to the change of the air pressure in the cavity is substantially constant. Said initial pressure adjusting unit may adjust the initial pressure within a range where a ratio of the volume change to the change of the air pressure in the cavity shifts substantially linear. Thereby, the conversion error can be reduced when converting the amount of atmospheric pressure change into the amount of volume change.

Said initial pressure adjusting unit may adjust the initial pressure to be substantially constant when measuring a subject for a plurality of times. Said initial pressure adjusting unit may adjust the initial pressure to be substantially constant when measuring a plurality of subjects. Thereby, since the conditions at the time of measurement can be kept constant, a measurement error can be reduced and the measurement data of good reproducibility can be obtained.

The measuring system may further include an indicating unit which indicates suitable respiratory movement to a subject according to the kind of data which should be measured. Timing may be planned with monitoring the subject's respiratory waveform, and directions may be taken out to the subject. Since anyone can measure the respiration function appropriately even if there is no assistance of a doctor and/or an assistant etc., a subject himself/herself can perform measurement at home, and the measurement can be performed even in a doctor-less village in an isolated island or a depopulated area, etc.

The measuring system may further include a condition input unit which receives an input of information about a measurement condition. The measuring system may further include a measurement control unit which controls said initial pressure adjusting unit or said indicating unit based on the measurement condition.

Said measurement control unit may determine the initial pressure which said initial pressure adjusting unit adjusts based on the measurement condition. For example, initial pressure may be changed by a male or by a woman, and also initial pressure may be changed by a subject who has a respiratory disease or by a healthy person. Initial pressure may be changed according to a subject's size of the chest, waist, and/or the corpulence degree, etc.; further initial pressure may be changed according to a subject's respiratory function.

Said measurement control unit may determine a content which said indicating unit indicates to a subject based on the measurement condition. For example, the direction content may be changed according to a subject's anamnesis.

The measuring system may further include a waveform generating unit for generating respiratory waveform data which shows respiratory state of a subject from the volume change near thorax which the first measuring unit detects, and the volume change near diaphragm which the second measuring unit detects. Said waveform generating unit may weight the volume change near thorax and the volume change near diaphragm with a predetermined ratio when generating the respiratory waveform data. Also the ratio with which volume change is edited may be determined according to the personal information, such as the abdomen contribution percentage, sex of the subject, and anamnesis of the subject, etc.

The measuring system may further include a calculating unit for calculating at least one respiratory function barometer among a lung capacity fraction, a forced expiratory curve, a forced lung capacity, a forced expiratory volume in one second, a forced expiratory rate in one second, a maximum mid-expiratory flow, a maximum ventilation volume, a flow volume curve, a peak expiratory flow rate, and a rate of abdomen contribution based on at least one of the volume change near thorax, the volume change near diaphragm and the respiratory waveform data. Said calculating unit may calculate the respiratory function barometer, converting at least one of the volume change near thorax, the volume change near diaphragm, and the respiratory waveform data into respiratory volume.

The measuring system may further include a waveform characteristic extracting unit which extracts a feature of waveform from at least one of the volume change near thorax, the volume change near diaphragm, and the respiratory waveform data. The feature of a differential function, a secondary differential function, and/or a third differential function and the like of each waveform may be extracted. The feature may be extracted by comparing with each waveform.

The measuring system may further include a respiratory tract state judging unit which judges state of constriction or blockage of a respiratory tract with reference to the respiratory waveform data, the forced expiration curve, or the flow volume curve. Said respiratory tract state judging unit may judge that a respiratory tract of a subject is constricted or blocked in the case where the respiratory waveform data, the forced expiratory curve, or the flow volume curve has a waveform showing an increase of the air pressure in the cavity when the subject starts expiration.

Although the waveform which shows the atmospheric pressure in a cavity decreases in connection with the expiration is measured in the case of a healthy person who does not have a constriction in a respiratory tract, the phenomenon which the atmospheric pressure, i.e., the chest data, or abdomen data tends to increase according to factors, such as restriction of the air current in the constriction portion at the time of the expiration or the abnormal movement of the respiratory muscle at the time of the expiration, has been observed by this inventor. Since such a phenomenon is characteristically seen for an asthmatic patient who has the condition of a respiratory tract constriction, the measurement result is useful to diagnose the respiratory tract constriction. Said respiratory tract state judging unit may judge a degree of constriction or blockage of the respiratory tract based on an aspect of increasing of the air pressure.

The measuring system may further include a database which stores a medical view correspondent with at least one of a feature of the waveform of the respiratory waveform data, a feature of the waveform of the volume change near thorax, a feature of the waveform of the volume change near diaphragm, a difference of the waveform between the volume change near thorax and the volume change near diaphragm, a respiratory function barometer, and a personal data of a subject.

The measuring system may further include a database referring unit which acquires the medical view for the subject with reference to said database. The measuring system may further include a display unit which displays the medical view.

Another embodiment according to the present invention relates to a measuring apparatus. This measuring apparatus is a measuring apparatus for measuring respiratory function characterized in that it includes: a sensing unit for sensing volume change of a measurement part of a subject sequent to respiratory movement; and a fixing unit for arranging said sensing unit near the measurement part; wherein said fixing unit has a belt-like form and can fix said sensing unit in a manner of impressing said sensing unit on the measurement part; and said sensing unit senses the volume change of the measurement part from change of pressure applied by said fixing unit and the measurement part.

Still another embodiment according to the present invention also relates to a measuring apparatus. This measuring apparatus is a measuring apparatus for measuring respiratory function characterized in that it includes: a first measuring unit for detecting volume change of a first measurement part of a subject sequent to respiratory movement; and a second measuring unit for detecting volume change of a second measurement part of the subject sequent to respiratory movement; said first measuring unit and said second measuring unit respectively include: a sensing unit for sensing the volume change of the measurement part; and a fixing unit for arranging said sensing unit near the measurement part; wherein said fixing unit has a belt-like form and can fix said sensing unit in a manner of impressing said sensing unit on the measurement part; and said sensing unit senses the volume change of the measurement part from change of pressure applied by said fixing unit and the measurement part.

Said sensing unit may have a cavity inside and includes a first connecting unit for sending gas which is in the cavity to a pressure sensor for measuring air pressure in the cavity. Said sensing unit may further include a second connecting unit for connecting with a pump for sending gas into the cavity. The first connecting unit and the second connecting unit may be a tube formed by a rubber, a resin, or the like.

The measuring apparatus may further include a control unit which includes said pressure sensor and said pump. Said control unit may further include a recording unit which records the air pressure in the cavity which said pressure sensor measures. Said recording unit may record the air pressure in the cavity, or data converted from the air pressure into the volume or respiratory volume in an external recording medium.

Said control unit may further include a transfer unit which transfers the air pressure in the cavity which said pressure sensor measures to an analyzing apparatus for analyzing respiratory function. The transfer unit may transfer the data using arbitrary communicating means of a cable or a radio, such as the internet, a cellar-phone network, or infrared ray.

The measuring apparatus may further include an initial pressure adjusting unit for adjusting the air pressure in the cavity to predetermined initial pressure by sending gas into the cavity before a measurement of respiratory function. Said initial pressure adjusting unit may adjust the initial pressure within a range where a ratio of the volume change to the change of the air pressure in the cavity is substantially constant. Said initial pressure adjusting unit may adjust the initial pressure within a range where a ratio of the volume change to the change of the air pressure in the cavity shifts substantially linear. Said initial pressure adjusting unit may adjust the initial pressure to be substantially constant when measuring a subject for a plurality of times. Said initial pressure adjusting unit may adjust the initial pressure to be substantially constant when measuring a plurality of subjects.

Still another embodiment according to the present invention relates to an analyzing apparatus. This analyzing apparatus is an analyzing apparatus for analyzing respiratory function of a subject characterized in that it includes: a measurement data acquiring unit which acquires respiratory function measurement data of the subject; and a calculating unit for calculating at least one respiratory function barometer among a lung capacity fraction, a forced expiratory curve, a forced lung capacity, a forced expiratory volume in one second, a forced expiratory rate in one second, a maximum mid-expiratory flow, a maximum ventilation volume, a flow volume curve, a peak expiratory flow rate, and a rate of abdomen contribution.

The respiratory function measurement data may include chest data acquired by measurement of volume change near thorax and abdomen data acquired by measurement of volume change near diaphragm; and said analyzing apparatus may further include a waveform generating unit which generates respiratory waveform data which shows respiratory state of a subject from the chest data and the abdomen data. Said waveform generating unit may weight the chest data near thorax and the abdomen data with a predetermined ratio when generating the respiratory waveform data.

The analyzing apparatus may further include a waveform characteristic extracting unit which extracts a feature of waveform from at least one of the volume change near thorax, the volume change near diaphragm, and the respiratory waveform data. The analyzing apparatus may further include a respiratory tract state judging unit which judges state of constriction or blockage of a respiratory tract with reference to the respiratory waveform data, the forced expiration curve, or the flow volume curve. Said respiratory tract state judging unit may judge that a respiratory tract of a subject is constricted or blocked in the case where the respiratory waveform data, the forced expiratory curve, or the flow volume curve has a waveform showing an increase of the chest data or the abdomen data of the subject when the subject starts expiration. Said respiratory tract state judging unit may judge a degree of constriction or blockage of the respiratory tract based on an aspect of increasing of the chest data or the abdomen data. The analyzing apparatus may further include a database referring unit which acquires a medical view for a subject with reference to a database which stores the medical view correspondent with at least one of a feature of the waveform of the respiratory waveform data, a feature of the waveform of the volume change near thorax, a feature of the waveform of the volume change near diaphragm, a difference of the waveform between the volume change near thorax and the volume change near diaphragm, a respiratory function barometer, and a personal data of the subject.

Still another embodiment according to the present invention relates to a computer program. This program makes a computer realize: a function of acquiring respiratory function measurement data of a subject; and a function of calculating at least one respiratory function barometer among a lung capacity fraction, a forced expiratory curve, a forced lung capacity, a forced expiratory volume in one second, a forced expiratory rate in one second, a maximum mid-expiratory flow, a maximum ventilation volume, a flow volume curve, a peak expiratory flow rate, and a rate of abdomen contribution.

The respiratory function measurement data may include chest data acquired by measurement of volume change near thorax and abdomen data acquired by measurement of volume change near diaphragm; and the program may further make a computer realize a function of generating respiratory waveform data which shows respiratory state of a subject from the chest data and the abdomen data. The respiratory waveform data may be generated by weighting the chest data near thorax and the abdomen data with a predetermined ratio.

The program may further make a computer realize a function of extracting a feature of waveform from at least one of the volume change near thorax, the volume change near diaphragm, and the respiratory waveform data. The program may further make a computer realize a function of judging state of constriction or blockage of a respiratory tract with reference to the respiratory waveform data, the forced expiration curve, or the flow volume curve. The program may judge that a respiratory tract of a subject is constricted or blocked in the case where the respiratory waveform data, the forced expiratory curve, or the flow volume curve has a waveform showing an increase of the chest data or the abdomen data of the subject when the subject starts expiration. The program may judge a degree of constriction or blockage of the respiratory tract based on an aspect of increasing of the chest data or the abdomen data. The program may further make a computer realize a function of acquiring a medical view for a subject with reference to a database which stores the medical view correspondent with at least one of a feature of the waveform of the respiratory waveform data, a feature of the waveform of the volume change near thorax, a feature of the waveform of the volume change near diaphragm, a difference of the waveform between the volume change near thorax and the volume change near diaphragm, a respiratory function barometer, and a personal data of the subject.

Still another embodiment according to the present invention relates to an analyzing server. This analyzing server is an analyzing server for analyzing respiratory function of a subject characterized in that it comprises: a database stored respiratory function measurement data and a medical view correspondingly; a receiving unit which receives a requirement of referring said database through a network; a measurement data acquiring unit which acquires the respiratory function measurement data of the subject; a database referring unit which acquires the medical view with reference to said database based on the respiratory function measurement data; and a transmitting unit which transmits the medical view through the network.

This analyzing server provides the database for leading a medical view from the data measured by above-mentioned measuring apparatus. The medical view can be acquired by accessing the analyzing server with a terminal of hospital, a clinic, and a rehabilitation facility, for example, and sending the respiratory function measurement data of the subject to the analyzing server. A business model can work out where the database is managed by the analyzing server 400 in the block without placing them at each terminal, and a user using the database is charged.

The respiratory function measurement data may include chest data acquired by measurement of volume change near thorax and abdomen data acquired by measurement of volume change near diaphragm. The respiratory function measurement data may be respiratory waveform data generated using the chest data and the abdomen data. The respiratory waveform data may be generated by weighting the chest data and the abdomen data with a predetermined ratio.

Still another embodiment according to the present invention relates to a rehabilitation assisting apparatus. This rehabilitation assisting apparatus is a rehabilitation assisting apparatus which assists rehabilitation of respiratory function characterized in that it comprises: a pressurizing unit which includes a pressurizing member for squeezing a body of a subject; and a fixing unit for arranging said pressurizing member to the body of the subject; wherein said fixing unit has a belt-like form and can fix said pressurizing member in a manner of impressing said pressurizing member on the body; and said pressurizing member has a bag-like form and squeezes the body with increasing volume thereof by introducing gas therein.

Said pressurizing member may be fixed so that said pressurizing member squeezes an expectoration part of the subject. Said pressurizing unit may have a plurality of said pressurizing members; and said rehabilitation assisting apparatus may further comprise a control unit which controls volume of gas inside said pressurizing members so that said pressurizing members squeeze the expectoration part. This performs the technique generally called "squeezing." As for rehabilitation, arbitrary rehabilitations such as breathing under pressurization load, and/or a exercise such as a walk, etc., may be carried out.

The rehabilitation assisting apparatus may comprise a plurality of said pressurizing units; and said pressurizing units may be arranged at least near thorax and near diaphragm of the subject. The rehabilitation assisting apparatus may further comprise a pressure sensor which measures air pressure inside said pressurizing member; and respiratory state of the subject may be measured by sensing the volume change near thorax or diaphragm from change of the air pressure measured by said pressure sensor. Thereby, the state of thoracic respiration and abdominal respiration can be independently measured like above-stated measurement equipment. Moreover, the respiratory function can be continuously measured after the rehabilitation, without removing the pressurizing units. Thus, this rehabilitation assisting apparatus has function of both the pressurization of the expectoration part and the function of respiratory measurement at the time of rehabilitation.

Still another embodiment according to the present invention relates to a measuring method. This method is a method of measuring respiratory function characterized in that it includes: a step of fixing a first measuring unit which includes a first sensing unit for sensing volume change of a first measurement part of a subject sequent to respiratory movement; and a first fixing unit for arranging said first sensing unit to the first measurement part, in such a manner that said first fixing unit pinches said first sensing unit between said first fixing unit and the first measurement part; a step of fixing a second measuring unit which includes a second sensing unit for sensing volume change of a second measurement part of the subject sequent to respiratory movement; and a second fixing unit for arranging said second sensing unit to the second measurement part, in such a manner that said second fixing unit pinches said second sensing unit between said second fixing unit and the second measurement part; a step of measuring simultaneously the volume change of the first measurement part sensed by said first sensing unit and the volume change of the second measurement part sensed by said second sensing unit.

The first measurement part may be near thorax and the second measurement part may be near diaphragm. Said first fixing unit and said second fixing unit may have belt-like forms; said first sensing unit and said second sensing unit may have bag-like forms; said step of fixing may fix said first fixing unit and said second fixing unit in such a manner that a body of the subject is wrapped around by said first fixing unit and said second fixing unit; and said step of measuring may measure the volume change by sensing air pressure inside said first sensing unit and said second sensing unit.

The method may further include, between said step of fixing and said step of measuring, a step of adjusting the air pressure inside said sensing unit to predetermined initial pressure by sending gas into said sensing unit; and said step of measuring may measure maintaining an amount of gas introduced into said sensing unit in said adjusting. The valve etc. may be prepared in each sensing unit so that the gas sent inside of the sensing unit may not leak out to the outside. Thereby, it can prevent the condition change during the measurement.

Still another embodiment according to the present invention relates to a rehabilitation assisting method. This method is a method of assisting rehabilitation of respiratory function characterized in that it includes: a step of equipping a subject with a pressurizing unit which comprises a pressurizing member for squeezing a body of the subject and a fixing unit for arranging the pressurizing member to the body; a step of squeezing the body of the subject by the pressurizing member; a rehabilitation step of making the subject perform respiratory movement under squeezing; a first measurement step of measuring respiratory state of the subject by sensing pressure applied to the pressurizing member while said rehabilitation step.

The fixing unit may have a belt-like form; the pressurizing member may have a bag-like form; said step of equipping may fix the pressurizing member in such a manner that the pressurizing member is pinched between the fixing unit and the body of the subject; said step of squeezing may squeeze the body with increasing volume of the pressurizing member by introducing gas into the pressurizing unit.

Said step of equipping may equip the subject with the pressurizing member so that the pressurizing member squeezes an expectoration part of the subject; said step of squeezing may squeeze the expectoration part by adjusting air pressure inside the pressurizing member.

The method may include after said rehabilitation step: a step of adjusting air pressure inside the pressurizing member to predetermined pressure; a second measurement step of measuring respiratory state of the subject after rehabilitation by sensing the air pressure inside the pressurizing member.

The method may further include a step of evaluating appropriate value of pressurizing to squeeze the body in said rehabilitation step based on the respiratory state of the subject measured in said first measurement step. Thereby, during the rehabilitation, the subject's respiratory condition can be monitored, and suitable pressurization can be applied.

The method may further include a step of evaluating effect of said rehabilitation step based on the respiratory state of the subject measured in said second measurement step. Since the subject's respiratory condition can be monitored immediately after starting of the rehabilitation, it is suitable for measuring the change of the respiratory state with the passage of time after the subject starts rehabilitation.

It is to be noted that any arbitrary combination of the above-described elements, and expressions changed between a method, an apparatus, a system, a recording medium, a computer program and so forth are all effective and encompassed by the present embodiments.

### BRIEF DESCRIPTION OF THE DRAWINGS

The above objects and other objects, features and advantages will be better understood when considered in view of the following detailed description of the preferred embodiments and accompanying drawings.
Fig. 1 is a figure showing a composition of a measuring system according to an embodiment.
Fig. 2(a) and 2(b) are figures schematically showing an appearance of a measuring unit.
Fig. 3 is a figure showing how a subject is equipped with the measuring unit.
Fig. 4(a), 4(b), and 4(c) are figures showing cross sections of a part equipped with the measuring unit.
Fig. 5 is a figure showing other example of a fixing unit.
Fig. 6 is a figure schematically showing an appearance of a control unit.
Fig. 7 is a flow chart which shows procedures of measuring respiratory function using the measuring system of the embodiment.
Fig. 8 is a flow chart which shows procedures of analyzing the respiratory function using the measuring system of the embodiment.
Fig. 9 is a figure showing personal information of subjects who went through a respiratory function inspection using the measuring system of the embodiment.
Fig. 10 is a figure showing change of inner pressure of a sensing unit with passage of time when measuring the lung volume fraction of a healthy person as the subject using the measuring system of the embodiment.
Fig. 11(a) and 11(b) are figures showing measurement results of the vital capacity of healthy persons as the subject using the measuring system of the embodiment and the Spirometer.
Fig. 12(a) and 12(b) are figures showing measurement results of the expiratory reserve volume of healthy persons as the subject using the measuring system of the embodiment and the Spirometer.
Fig. 13(a) and 13(b) are figures showing measurement results of the inspiratory reserve volume of healthy persons as the subject using the measuring system of the embodiment and the Spirometer.
Fig. 14(a) and 14(b) are figures showing measurement results of the inspiratory capacity of healthy persons as the subject using the measuring system of the embodiment and the Spirometer.
Fig. 15(a) and 15(b) are figures showing measurement results of the tidal volume of healthy persons as the subject using the measuring system of the embodiment and the Spirometer.
Fig. 16 is a figure showing change of inner pressure of a sensing unit with passage of time when measuring the forced expiratory curve of a healthy person as the subject using the measuring system of the embodiment.
Fig. 17(a), 17(b) and 17(c) are figures showing the flow volume curve of a healthy person as the subject measured by the measuring system of the embodiment.
Fig. 18(a) and 18(b) are figures showing measurement results of the forced expiratory capacity of healthy persons as the subject using the measuring system of the embodiment and the Spirometer.

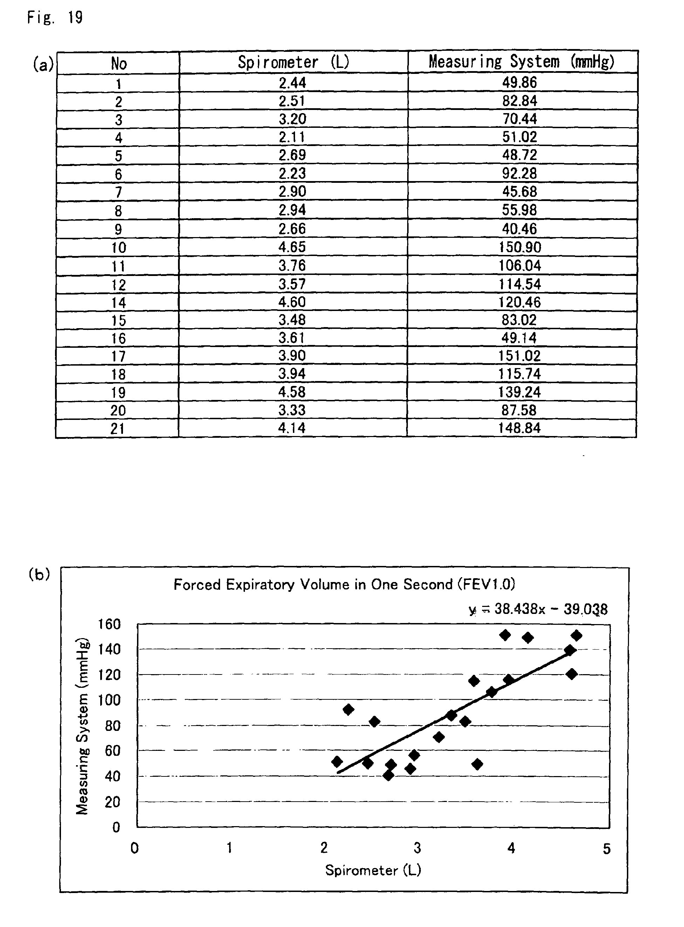

Fig. 19(a) and 19(b) are figures showing measurement results of the forced expiratory volume in one second of healthy persons as the subject using the measuring system of the embodiment and the Spirometer.
Fig. 20(a) and 20(b) are figures showing measurement results of the peak expiratory flow rate of healthy persons as the subject using the measuring system of the embodiment and the Spirometer.
Fig. 21 is a figure showing changes of inner pressure of a sensing unit with passage of time when measuring the ventilation volume at resting of a healthy person as the subject using the measuring system of the embodiment.
Fig. 22(a) and 22(b) are figures showing measurement results of the minutes volume of healthy persons as the subject using the measuring system of the embodiment and the Spirometer.
Fig. 23(a) and 23(b) are figures showing measurement results of the tidal volume of healthy persons as the subject using the measuring system of the embodiment and the Spirometer.
Fig. 24(a) and 24(b) are figures showing measurement results of the peak interval of waveform of healthy persons as the subject using the measuring system of the embodiment and the Spirometer.
Fig. 25 is a figure showing a relation between the chest data and the abdomen data when measuring the ventilation volume at resting of a healthy person as the subject using the measuring system of the embodiment.
Fig. 26 is a figure showing a relation between the chest data and the abdomen data when measuring the forced expiratory curve of a healthy person as the subject using the measuring system of the embodiment.
Fig. 27(a) and 27(b) are figures showing measurement results of the vital capacity of patients who have respiratory disease as the subject using the measuring system of the embodiment and the Spirometer.
Fig. 28(a) and 28(b) are figures showing measurement results of the expiratory reserve volume of patients who have respiratory disease as the subject using the measuring system of the embodiment and the Spirometer.
Fig. 29(a) and 29(b) are figures showing measurement results of the inspiratory reserve volume of patients who have respiratory disease as the subject using the measuring system of the embodiment and the Spirometer.
Fig. 30(a) and 30(b) are figures showing measurement results of the inspiratory capacity of patients who have respiratory disease as the subject using the measuring system of the embodiment and the Spirometer.
Fig. 31(a) and 31(b) are figures showing measurement results of the tidal volume of patients who have respiratory disease as the subject using the measuring system of the embodiment and the Spirometer.
Fig. 32(a) and 32(b) are figures showing measurement results of the forced expiratory capacity of patients who have respiratory disease as the subject using the measuring system of the embodiment and the Spirometer.
Fig. 33(a) and 33(b) are figures showing measurement results of the peak expiratory flow rate of patients who have respiratory disease as the subject using the measuring system of the embodiment and the Spirometer.
Fig. 34(a) and 34(b) are figures showing measurement results of the minutes volume of patients who have respiratory disease as the subject using the measuring system of the embodiment and the Spirometer.
Fig. 35(a) and 35(b) are figures showing measurement results of the tidal volume of patients who have respiratory disease as the subject using the measuring system of the embodiment and the Spirometer.
Fig. 36(a) and 36(b) are figures showing measurement results of the peak interval of waveform of patients who have respiratory disease as the subject using the measuring system of the embodiment and the Spirometer.
Fig. 37 is a figure showing change of inner pressure of a sensing unit with passage of time when measuring the forced expiratory curve of a patient who has respiratory disease as the subject using the measuring system of the embodiment.
Fig. 38 is a figure showing the flow volume curve of a patient who has respiratory disease as the subject measured by the measuring system of the embodiment.
Fig. 39 is a figure showing a relation between the value of rising of pressure inside the sensing unit and the peak expiratory flow rate at forced expiration.
Fig. 40 is a figure showing a structure of an analyzing server according to the embodiment and a terminal which accesses the analyzing server.
Fig. 41(a), 41(b), 41(c), 41(d), and 41(e) are the figures showing samples of usage of a rehabilitation assisting apparatus according to the embodiment.
Fig. 42 is a flow chart which shows a rehabilitation assisting method according to the embodiment.

### BEST MODE FOR CARRYING OUT THE INVENTION

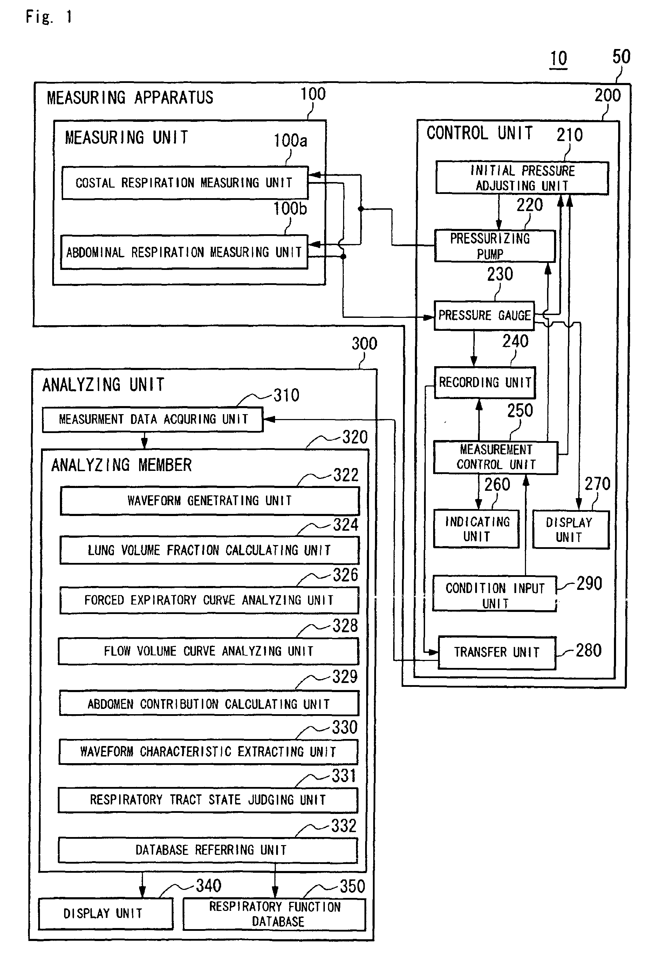

Fig. 1 is a composition figure of the measuring system 10 according to an embodiment of the present invention. The measuring system 10 mainly includes a measuring apparatus 50 equipping with a measuring unit 100 for measuring a subject's respiratory condition and a control unit 200 which acquires an output from the measuring unit 100, and an analyzing unit 300 for analyzing the output which the control unit 200 acquires. In Fig. 1, a part of structure of the control unit 200 and the analyzing unit 300 can be realized, in terms of hardware, by a CPU, a memory, memory-loaded programs or the like, but drawn and described here are function blocks that are realized in cooperation with those. Thus, it is understood by those skilled in the art that these functional blocks can be realized in a variety of forms by hardware only, software only or the combination thereof.

The measuring unit 100 includes a costal respiration measuring unit 100a for measuring a volume change near thorax as an example of a first measuring unit and an abdominal respiration measuring unit 100b for measuring a volume change near the diaphragm as an example of a second measuring unit. The costal respiration measuring unit 100a and the abdominal respiration measuring unit 100b detect the volume changes of the measurement parts by changes of the atmospheric pressure inside a sensing unit, respectively. Thereby, the measuring system 10 of the present embodiment can measure the data corresponding to the costal respiration and the data corresponding to the abdominal respiration independently. Moreover, reproducibility is good and it is a big merit that reliable data is obtained also quantitatively.

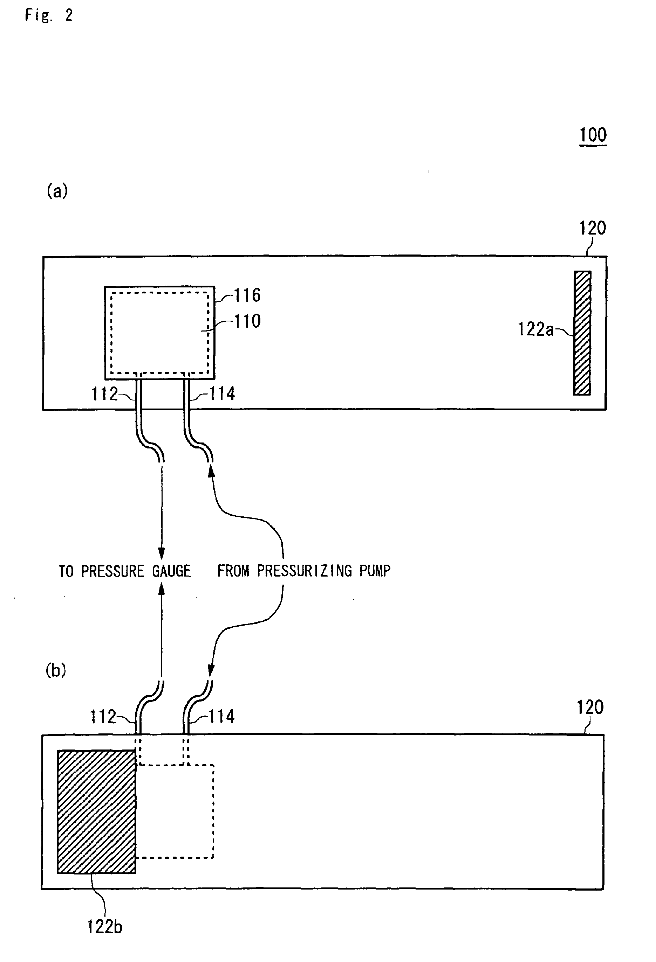

Fig. 2(a) and 2(b) schematically show the appearance of the measuring unit 100. Fig. 2(a) is a figure seeing the measuring unit 100 from the side which touches a subject's body and the Fig. 2(b) is a figure seeing the measuring unit 100 from the reverse-side. The measuring unit 100 includes the sensing unit 110 for detecting a volume change of the measurement part accompanying respiratory action of a subject, and a fixing unit 120 for arranging the sensing unit 110 near the measurement part. The sensing unit 110 has a bag-like form with a cave inside, and detects a volume change of the measurement part with change of the atmospheric pressure inside. The sensing unit 110 is equipped with the tube 114 for sending gas inside from the pressure pump 220 as an example of a first connection member and a tube 112 for sending gas to a pressure gauge as an example of a second connection member. The sensing unit 110 is contained in the case 116 made in the fixing unit 120.

The fixing unit 120 has a belt-like form and is wrapped around the subject's body, arranging the sensing unit 110 to the measurement part. Soft Materials, such as soft cloth, may be used for the fixing unit 120 so that it may be easily wrapped around the subject's body. Moreover, the material with little elasticity, such as canvas cloth, may be used so that it may not expand greatly by respiratory motion of a subject. The adhesion members 122a and 122b for attaching both ends of the fixing unit 120, when the fixing unit 120 is wrapped around the subject's body, are formed in the fixing unit 120. The adhesion members 122a and 122b may be a zipper or a plane fastener, etc.

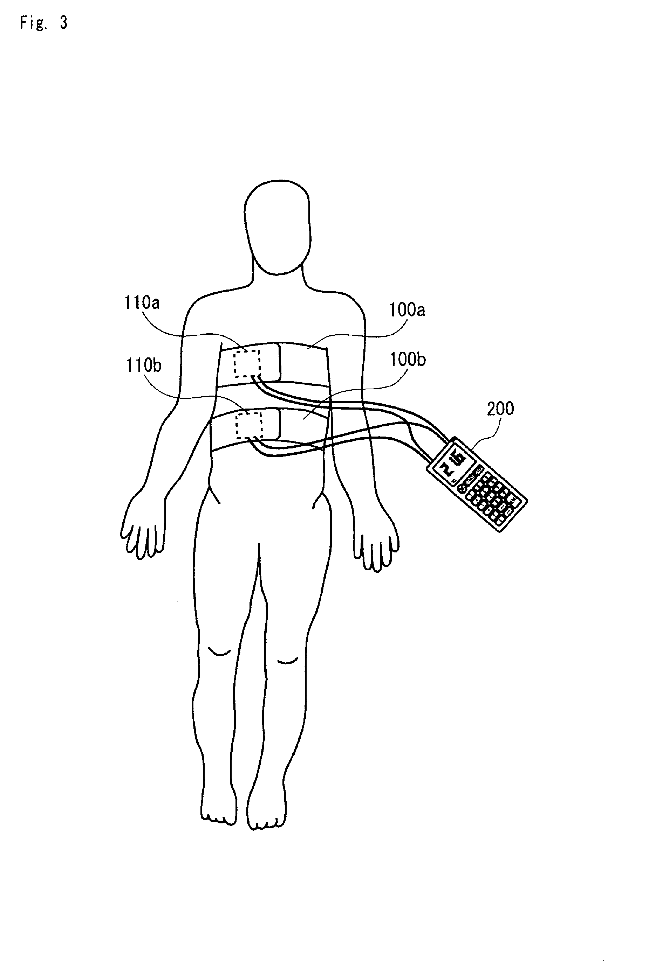

Fig. 3 shows how the subject is equipped with the costal respiration measuring unit 100a and the abdominal respiration measuring unit 100b. The costal respiration measuring unit 100a is equipped around the subject's chest. The abdominal respiration measuring unit 100b is equipped around subject's diaphragm. When equipping a subject with the measuring unit 100, the sensing unit 110 is first stuck to the measurement part, and the fixing unit is wrapped around the subject's body firmly. Since there is sometimes a right-and-left difference in muscular power of the respiratory muscles, the position of the sensing unit 110 may be determined considering the subject's dominant hand. For example, the position in which the sensing unit 110 is fixed may be unified to be the dominant hand side or the contrary side of the subject. The position in which the sensing unit 110 is fixed may be unified to be the subject's left half or right half of the body.

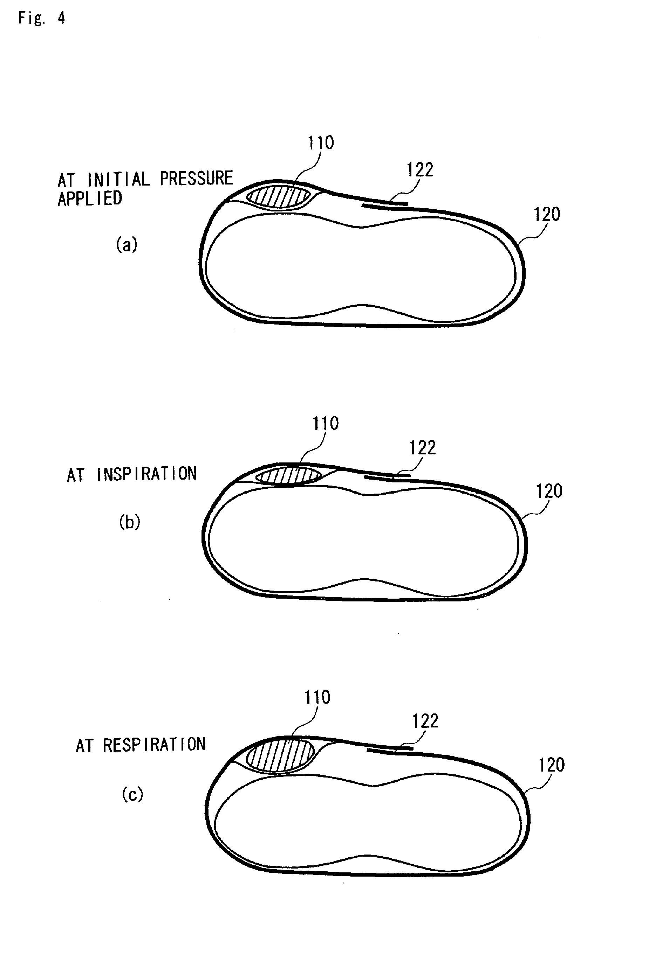

Fig. 4(a), 4(b), and 4(c) show cross sections of a subject equipped with the measuring unit 100. Fig. 4(a) shows a situation when an initial pressurization is performed by sending gas inside the sensing unit 110 from the pressurization pump 220 after the subject is equipped with the measuring unit 100. The Gas is sent into the inside of the sensing unit 110, and empty space is made between the fixing unit 120 and the measurement part. Since it is necessary to change into the state where a certain amount of pressure is impressed to the sensing unit also at the time of the maximum inspiration in order to measure the volume change of the measurement part correctly at the time of inspiration, the initial pressurization is set in this way. Moreover, it is meaningful for keeping measurement conditions constant by standardizing an initial pressurization value. Fig. 4(b) shows a situation when a subject performs inspiration action. When a subject inhales air, the volume near the thorax and the diaphragm of the subject increase, then, the sensing unit 110 is compressed between the subject's body and the fixing unit, therefore internal atmospheric pressure becomes high. Fig. 4(c) shows a situation when the subject performs expiration action. When the subject exhales, the atmospheric pressure inside the sensing unit 110 becomes low because the volume near thorax and diaphragm of the subject may decrease, and empty space between the subject's body and the fixing unit spreads out. Thus, the volume change near thorax and diaphragm can be detected with the change of inner pressure of the sensing unit 110.

When using this measuring system as rehabilitation assisting apparatus, the sensing unit 110 can function as a pressurization part. Namely, a subject's body is pressed with the predetermined load by sending gas inside of the sensing unit 110, and making the volume of the sensing unit 110 increase. A subject is able to perform rehabilitation while breathing under load. At this time, the measuring unit 100 may be equipped pressing the expectoration part of a subject by the sensing unit 110, and the rehabilitation is performed using the squeezing method.

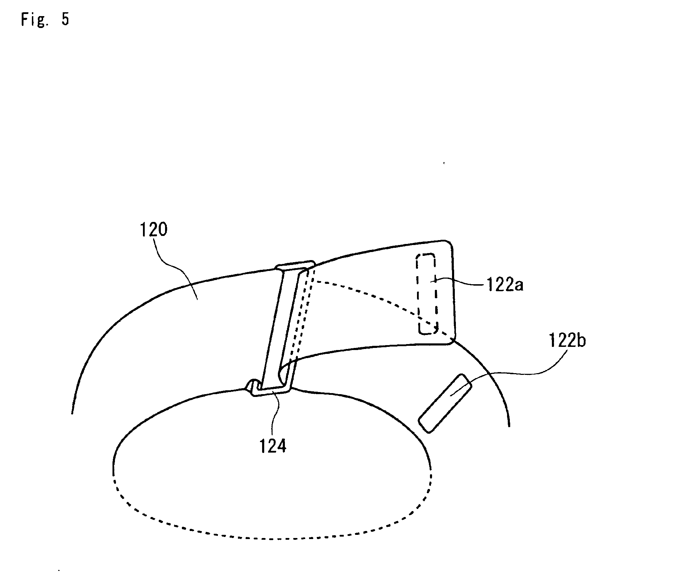

Fig. 5 shows another example of the fixing unit 120. In the example of Fig. 5, a hook 124 is formed at one end of the fixing unit 120, which lets another end of the fixing unit 120 pass through. At the time of wearing, another end is passed through the hook 124, and fix the adhesion member 122 after pulling the end fully to make it stick. The adhesion member 122 may be a zipper or a plane fastener, etc. According to the measuring unit 100 shown in Fig. 5, even if it is the case when a subject itself equips with the measuring unit 100, the measuring unit 100 can be fixed appropriately and easily.

Now, going back to Fig. 1 and the explanation of the control unit 200 and the analyzing unit 300 is continued. The control unit 200 includes an initial pressure adjusting unit 210, a pressurization pump 220, a pressure gauge 230, a recording unit 240, the measurement control unit 250, a indicating unit 260, a display unit 270, a transfer unit 280, and a condition input unit 290.

The initial pressure adjusting unit 210 controls the pressurization pump 220 to send gas inside of the sensing unit 110 and adjust the pressure to the predetermined initial value before measurement of the respiratory function. The initial pressure may be set up within the range in which the ratio between the volume change of the measurement part and the pressure change in the sensing unit 110 are approximately same, or may be set up within the range in which the ratio between the volume change of the measurement part and the pressure change in the sensing unit 110 is linear. If the initial pressure is too large, a burden will be given to a subject and there is a possibility that a respiratory state cannot be measured correctly. If the initial pressure is too small, there is a possibility that the pressure change in the sensing unit 110 may not follow accordingly to the volume change of the measurement part. What is necessary is just to adjust the initial pressure to the range in which the pressure change in the sensing unit 110 can be converted properly to the volume change of the measurement part, or respiratory volume at the time of breathing.

The initial pressure adjusting unit 210 may adjust the initial pressure to the constant value when measuring a subject for multiple times. With this, the measurement conditions can be maintained constantly for multiple measurements of the same subject, and the measurement results can be compared with each other and examined quantitatively. The initial pressure adjusting unit 210 may adjust the initial pressure to the constant value when measuring multiple subjects. Thereby, the measurement conditions can be kept constant and the measurement results can be compared with each other and examined quantitatively. Thus, since conditions can be unified when measuring by the initial pressure adjusting unit 210, the data obtained are with high reproducibility and reliable. The initial pressure may be determined based on a subject's sex, the rate of body fat, a chest circumference, a waist, an anamnesis, a lung capacity, the size of the sensing unit 110, or the like.

After the initial pressure adjusting unit 210 sends gas into the sensing unit 110, the measurement is performed maintaining the quantity of the gas inside the sensing unit 110. Thereby, the conditions can be kept constant while the measurement is performed. A valve may be attached to the sensing unit 110, so that gas once sent in would not flow outside.

The pressurization pump 220 sends gas inside the sensing unit 110, so that the pressure in the sensing unit 110 may become predetermined initial pressure. In the case of using this system as rehabilitation assisting apparatus, gas is sent inside of the sensing unit 110 by the pressurization pump 220 until it becomes predetermined load pressure.

The pressure gauge 230 as an example of the pressure sensor measures the atmospheric pressure inside the sensing unit 110. Arbitrary known pressure gauge or pressure sensor may be used as the pressure gauge 230. In the present embodiment, a pressure sensor that converts the pressure into a voltage signal by PIEZO resistive element is used.

The recording unit 240 samples the measurement data which the pressure gauge 230 measures in a predetermined sampling frequency, and records them as the respiratory waveform data. The recording unit 240 may record the measurement data in a memory inside the control unit 200, and/or may record them in a recording media, such as a floppy disk, CD-ROM, MO, and the like. Moreover, the recording unit 240 may not be comprised, and the measurement data may be transmitted directly to the analyzing unit 300. In the present embodiment, the voltage signals which the pressure gauge 230 measures are recorded in the memory inside the recording unit 240, and are transmitted to the analyzing unit 300 by the transfer unit 280. The voltage signals may be converted into a digital signal with A/D converter if necessary. Moreover, the voltage signals may be converted into pressure values and recorded or otherwise may be recorded after converting them into respiratory volume or respiration rate using a predetermined conversion formula.

The condition input unit 290 receives the information concerning measurement conditions from a subject or a measurer. For example, the condition input unit 290 may receive information, such as the kind of data which should be measured, a subject's sex, age, height, weight, chest circumference, waist size, a rate of body fat, and private information like about anamneses, measurement day, the number of times of measurement, temperature, atmospheric pressure, and humidity, etc. The information may be recorded by recording unit 240 together with measurement data and/or may be used for control of the measurement control unit 250 or analysis of the analyzing unit 300, if necessary.

The measurement control unit 250 controls at least one of the initial pressure adjusting unit 210, the pressurization pump 220, the recording unit 240, and the indicating unit 260 to perform the measurement according to the kind of data which should be measured. The measurement control unit 250 may have a table for determining contents, procedure , or the like of the measurement based on the conditions which the condition input unit 290 receives. For example, a subject's sex and initial pressure may be stored correspondingly in the table, or the kind of data which should be measured and the sampling frequency of the data at the time of recording may be stored correspondingly in the table. And, the measurement may be controlled referring this table.

Moreover, the measurement procedure suitable for the kind of data which should be measured may be indicated to a subject through the indicating unit 260. At this time, the contents of directions may be determined based on the measurement conditions which is received by the condition input unit 290. For example, when the forced vital capacity FVC is measured, the indicating unit 260 may indicate to a subject while monitoring the measurement values by the pressure gauge 230, to make the subject breathe in until reaching the maximum inspiration level, saying "Please continue inhaling until it becomes impossible to breathe", until the measurement value is stabilized at the constant value, and saying "Please breathe out at a one stretch", when it is judged that the measurement value is stabilized at the constant value. Thus, a suitable measurement procedure is given to a subject while the subject's respiratory condition is being observed. In order a suitable measurement is performed, the respiratory waveform used as a standard may be stored, and a timing of issuing the direction to the subject may be planned comparing the standard waveform to the subject's respiratory waveform.

When using this measuring system 10 as the rehabilitation assisting apparatus, the measurement control unit 250 may control a rehabilitation program. The rehabilitation programs may be saved beforehand in the measurement control unit 250, and may be chosen based on the conditions received by the condition input unit 290, and/or a rehabilitation program may be inputted by the condition input unit 290. At this time, the procedure of the rehabilitation may be indicated to a patient by the indicating unit 260. For example, when a patient requires the respiratory rehabilitation under pressurization, the patient receives pressure to the predetermined point with the pressurization pump 220, and performs rehabilitation of breathing for predetermined time under pressurization.

The indicating unit 260 indicates suitable respiratory operations to a subject according to the kind of data which should be measured. The indicating unit 260 may output the directions transmitted from the measurement control unit 250 to a subject, or may judge the necessary directions. Directions may be outputted by the display unit 270 as visual information, and/or may be outputted as sound information with a speaker etc.

The display unit 270 displays information such as the measured value by the pressure gauge 230, and classification of the data at a time of measuring. The display unit 270 may be used as an interface when receiving information from a measurer by the condition input unit 290.

The transfer unit 280 transfers the measured data to the analyzing unit 300. The transfer unit 280 may transfer measurement data to the analyzing unit 300 through the cable which connects the control unit 200 and the analyzing unit 300, and/or may transfer it using the communication means of cables, such as the telephone network, the cellular-phone network, the Internet, infrared rays, and the radio wave, or wireless radio. When the recording unit 240 records data in a recording medium, the transfer unit 280 may be not necessary since the data are sent to the analyzing unit 300 through the recording medium.

The analyzing unit 300 includes a measurement data acquiring unit 310, an analyzing member 320, a display unit 340, and a respiratory function database 350. The measurement data acquiring unit 310 acquires the data measured by the measuring unit 100 and the control unit 200. The analyzing member 320 includes a waveform generating unit 322, a lung volume fraction calculating unit 324, a forced expiratory curve analyzing unit 326, a flow volume curve analyzing unit 328, an abdomen contribution calculating unit 329, a waveform characteristic extracting unit 330, a respiratory tract state judging unit 331, and a database referring unit 332. The analyzing unit 300 may function as analyzing apparatus 300 independently apart from the measuring apparatus 50. The analyzing unit 300 and the analyzing apparatus 300 may be realized by an ordinary computer.

The measurement data acquiring unit 310 acquires measurement data from the control unit 200. The measurement data contains the chest data obtained by measurement of the volume changes near thorax, and the abdomen data obtained by measurement of the volume changes near diaphragm. These data may be expressed by the atmospheric pressure of sensing unit 110 inside, and/or may be converted into the volume changes of the measurement part, or respiratory volume at the time of breathing.

The waveform generating unit 322 generates respiratory waveform data from the measurement data which the measurement data acquiring unit 310 acquires. In this system, the respiratory waveform data is generated from the chest data obtained by measurement of the volume changes near thorax, and the abdomen data obtained by measurement of the volume changes near diaphragm. At this time, the chest data and the abdomen data may be weighted by the predetermined ratio, and the respiratory waveform data may be generated. As for the weighing ratio, it may be determined according to the rate of abdomen contribution, personal information such as sex of a subject, or the anamneses of the subject, etc. The waveform generating unit 322 may remove the data dispersed extremely, or the portion considered that the movement of the subject during measurement might affect data when the waveform generating unit 322 is generating the respiratory waveform data. The respiratory waveform curve may be smoothed with a spline function, a Bezier function, etc. When a difference is recognized in the peak position of the chest data and the abdomen data, the peak position may be adjusted to generate the respiratory waveform data.

The lung volume fraction calculating unit 324 as an example of the calculating unit computes the lung volume fraction based on the respiratory waveform data generated in the waveform generating unit 322. The forced expiratory curve analyzing unit 326 as an example of the calculating unit computes forced lung capacity, and volume of 1 second, etc. based on the respiratory waveform data shaped by the waveform generating unit 322. The flow volume curve analyzing unit 328 as an example of the calculating unit computes the maximum rate of the expiration flow etc. based on the flow volume curve which is obtained by differentiating the forced expiratory curve. The abdomen contribution calculating unit 329 as an example of the calculating unit computes the abdomen contribution rate from the chest data and the abdomen data. Each value may be computed from the chest data, the abdomen data, and respiratory waveform data, respectively. Each detailed calculating method is explained in the explanation of the first experiment to the fourth experiment.

The waveform characteristic extracting unit 330 extracts the characteristic features of the waveform of respiratory waveform data, chest data, and/or abdomen data. For example, such a characteristic feature that there is a gap in the peak position of the respiratory waveform of chest data and abdomen data, a feature that the peak of flow volume curve is flat, or a feature that a flat portion appears after the peak of flow volume curve, etc. The waveform features which the waveform extracting unit 330 should extract may be stored beforehand, and the features of the waveform may be extracted by judging whether a subject's respiratory waveform data agree with the stored features.

The respiratory tract state judging unit 331 judges a constriction or a blockage of a respiratory tract referring to a respiratory waveform data, a forced expiratory curve, and/or a flow volume curve. The respiratory tract state judging unit 331 may judge that the subject's respiratory tract is either constricted or blocked in the case the respiratory waveform data, the forced expiration curve, and the flow volume curve show a waveform that reveals an increase of inside pressure of the sensing unit 110 equipped near thorax or near diaphragm, when a subject starts exhaling. Although the waveform which shows the atmospheric pressure in a cave decreases in connection with exhaling in the case of a healthy person who does not have a constriction in the respiratory tract, in the case of a patient whose respiratory tract is constricted, the phenomenon which the atmospheric pressure in the cave, i.e., the chest data, or abdomen data increases because of factors, such as restriction of the air current in the constriction portion at the time of exhaling or the abnormal movement of the respiratory muscles at the time of exhaling, has been observed by the inventors of the present invention. Since such phenomena are characteristic of the asthmatic patients who have conditions of the respiratory tract constriction, the respiratory tract state judging unit 331 may judge that the subject who shows such measurement results is doubtful of having the respiratory tract constriction. The details of the phenomena are considered based on the results of the fifth experiment mentioned later. The respiratory tract state judging unit 331 may judge the degree of constriction or blockage of a respiratory tract based on the increasing situation of the atmospheric pressure inside the sensing unit 110 at the time of exhaling. For example, when the pressure increase is steep, the amount of pressure increase is large, or time until the increased pressure returns is long, the degree of a blockage of a respiratory tract is high. The degree of a blockage of respiratory tract may be judged by considering inclination of forced expiration curve, high order differentiation coefficient, an integration value, form, the height of a peak, etc. to be an index. The degree of constriction or a blockage of a respiratory tract may be judged with analyzing the respiratory waveform at the time of exhaling hydrodynamically.

The database referring unit 332 acquires a medical view based on the lung volume fraction, the forced lung capacity, the forced expiratory volume one second, the peak expiratory flow rate, the respiratory waveform, the forced expiratory curve, a flow volume curve, and the rate of abdomen contribution, the feature of the respiratory waveform extracted by the waveform characteristic extracting unit 330, etc. referring to the respiratory function database 350. At this time, further information, such as the subject's sex, age, height, weight, body fat, chest circumference, waist size, and anamneses may be taken into the reference.

The display unit 340 displays information, such as the measured chest data, the abdomen data, and/or the respiratory waveform data, the computed respiratory function barometer, and the medical view acquired from the respiratory function database 350, on a display screen such as a liquid crystal display.

The respiratory function database 350 stores information, such as respiratory function barometers of the lung volume fraction, the forced lung capacity, the forced expiratory volume 1 second, the peak expiratory flow rate, the respiratory waveform, the forced expiratory curve, the flow volume curve, and the rate of abdomen contribution, and the features of the respiratory waveform, correspondent with the medical views. At this time, subject's sex, age, height, weight, body fat, anamneses, etc. may be taken into consideration. For example, the feature "the peak of a flow volume curve is flat," and the medical view "there is a doubt of constriction in upper respiratory tract" may be saved correspondently. The medical views may not only be the name of a respiratory disease presumed from the measurement results, but also be the conditions of respiratory organs, respiratory muscles, state of other organs, grade of the disease, as well as a proposal of suitable rehabilitation program and exercise, etc.

In Fig. 1, although the measuring unit 100 and the control unit 200 are collectively shown as the measuring apparatus 50, they do not need to be constituted in one set. Moreover, each composition component may be comprised in other units. For example, the pressurization pump 220, the pressure gauge 230, the recording unit 240, etc. may be placed in the measuring unit 100 side, as well as the condition input unit 290 and the display unit 270, etc. may be formed in the analyzing unit 300 side. The control unit 200 and the analyzing unit 300 may be constituted in one set. Thus, combinations of the components are highly flexible, which will be understood by those skilled in the art. The respiratory function database 350 may be installed in the external server etc., and accessed through a communication means such as a network.

The measuring apparatus 50 and the analyzing apparatus 300 may be equipped in different places. At this time, the data measured by the measuring apparatus 50 may be provided to the analyzing apparatus 300 by an external memory media, such as a floppy disk, CD-ROM, and MO, or through a network. Thus the measurement data can be analyzed suppose a subject lives in a doctor-less village or a depopulated area such as an isolated island and the analyzing apparatus is not available in the neighborhood. Moreover, for the measuring apparatus 50 of the present embodiment, the measuring unit 100 can be equipped comparatively easily, the subject can measure by himself/herself or with the help of a family etc. If a subject owns the measuring apparatus 50 at his/her house, it also is possible for the subject to transfer the measured data to a hospital and to acquire a doctor's view. Therefore, even if the subject is difficult to go out, it is possible for the subject to receive appropriate diagnosis from a doctor. Moreover, for example, the grade of the blockage situation or condition of the respiratory tract of a patient of sleep apnea syndrome (SAS) can be diagnosed by is equipped with this measuring unit 100 during sleep at a house etc., and or measurement of the respiratory state at the time of sleep can be made continuously.

When the measuring apparatus 50 of the present embodiment is used as the rehabilitation assisting apparatus, a value of a load given to a subject during rehabilitation can be evaluated appropriately as the respiratory state in rehabilitation can be monitored. Moreover, the respiratory function of the subject can be inspected continuously without removing the measuring unit 100 after the rehabilitation, and the effect of the rehabilitation can be appropriately evaluated. Furthermore, because a subject can operate the measuring unit 100 easily by himself/herself, he/she can perform rehabilitation even in the environment where the assistance of a doctor or a physiotherapist cannot be given. For example, a subject can perform rehabilitation at home, and receive a diagnosis from a doctor by transferring the measurement data during and after rehabilitation.

Furthermore, the measuring apparatus 50 of the present embodiment can be used not only in the medical area but also in various places. For instance, the measuring apparatus 50 can be equipped in a sport facility, and then a user may enable it to check a respiratory state before exercise and after exercise. Moreover, the respiratory state can also be monitored during an aerobics performance. Thus, the measuring system 10 and the measuring apparatus 50 of the present embodiment are convenient to carry, and wearing and operation are also easy, as well as they may be used by various users in various places.

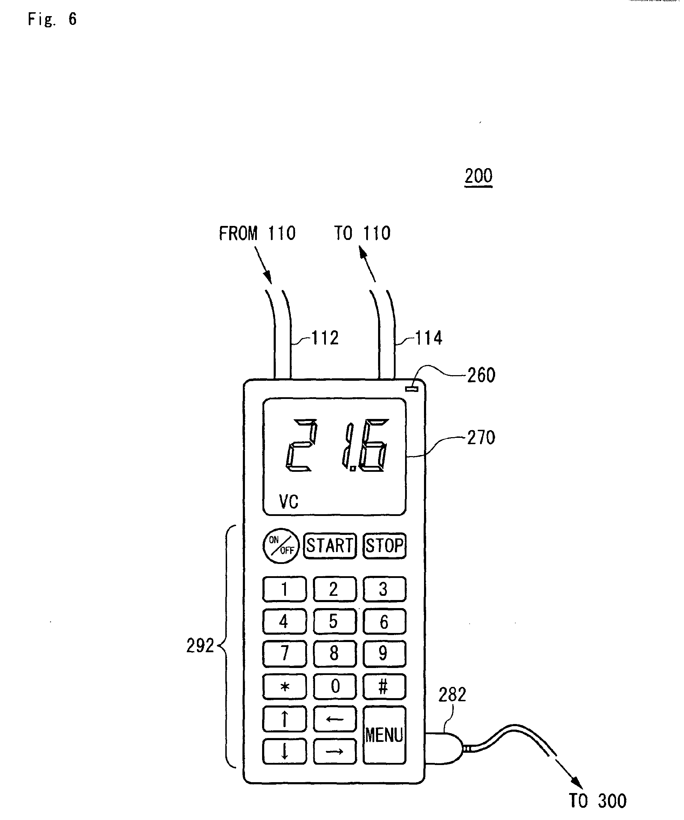

Fig. 6 schematically shows the appearance of the control unit 200. A speaker as an example of the indicating unit 260, a liquid crystal display unit as an example of the display unit 270, an input interface 292 for inputting conditions into the condition input unit 290, and a cable 282 for transferring the measurement data to the analyzing unit 300 by the transfer unit 280 are attached at the external surface of the control unit 200. Moreover, a tube 114 for the pressurization pump 220 to send gas inside of the sensing unit 110 and a tube 112 for measuring the inner pressure of the sensing unit 110 are connected.

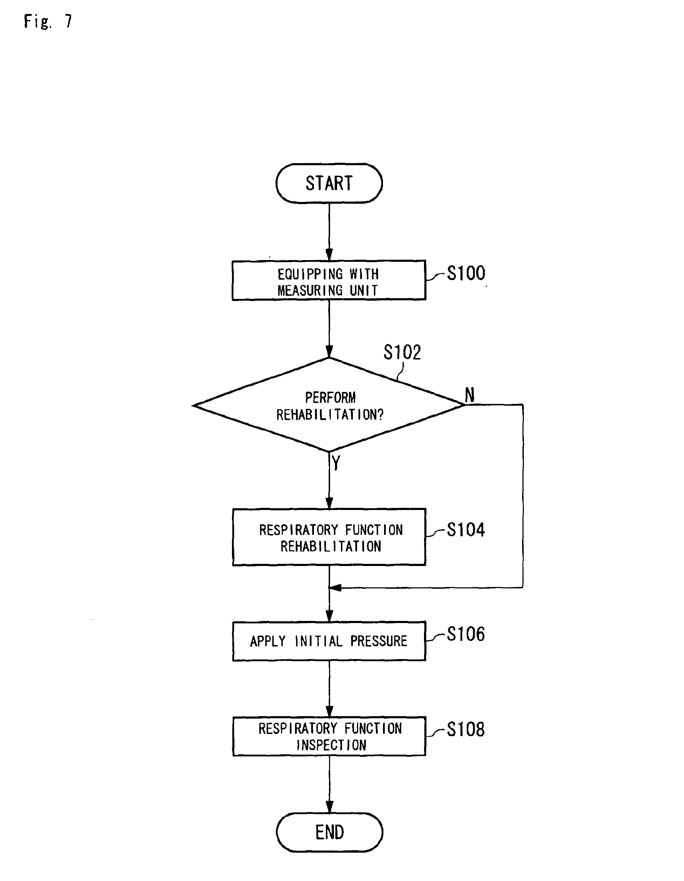

Fig. 7 shows the procedure for measuring the respiratory function using the measuring system 10 of the present embodiment. First, a subject is equipped with the costal respiration measuring unit 100a and the abdominal respiration measuring unit 100b (S100). Here, in the case rehabilitation is performed (Y of S102), the respiratory function rehabilitation are carried out (S104), and the respiratory function inspection is followed. In the case rehabilitation is not performed (N of S102), S104 is skipped. Prior to the respiratory function inspection, the initial pressurization is performed (S106) by sending gas inside of the sensing unit 110 using the pressurization pump 220. Then measurement of the respiratory function is carried out maintaining the amount of gas inside the sensing unit 110 (S108) .

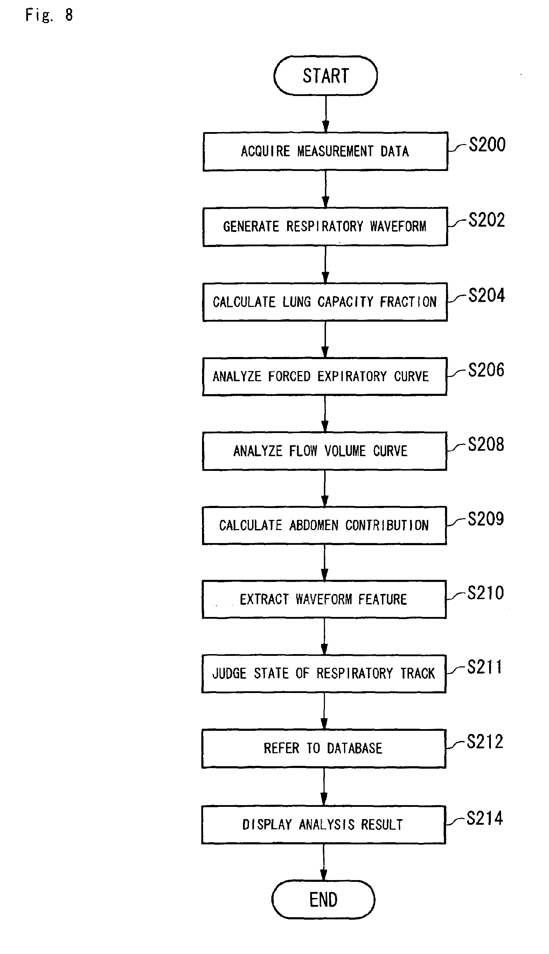

Fig. 8 shows the procedure for analyzing the respiratory function using the measuring system 10 of the present embodiment. First, the measurement data acquiring unit 310 acquires measurement data (S200). Then, the waveform generating unit 322 generates the respiratory waveform data from the measurement data (S202). Then, the lung volume fraction calculating unit 324 computes the lung volume fraction (S204), the forced expiration curve analyzing unit 326 computes the forced lung capacity and the forced expiratory volume 1 second, etc. (S206), the flow volume curve analyzing unit 328 computes the peak expiratory flow rate etc. (S208), and the abdomen contribution calculating unit 329 computes the rate of abdomen contribution etc. (S209). Then the waveform characteristic extracting unit 330 extracts the characteristic features of the respiratory waveform (S210). Furthermore, the respiratory tract state judging unit 331 judges the state of constriction or blockage of the respiratory tract with reference to the respiratory waveform data, the forced expiration curve, or the flow volume curve (S211). Further the database referring unit 332 acquires a medical view for the subject referring to the respiratory function database 350 based on the data, such as the feature of the lung volume fraction, the forced lung capacity, the peak expiratory flow rate, the rate of abdomen contribution, and the respiratory waveform, (S212). Finally, the analysis result is displayed on the display unit 340 (S214).

In the above, the composition and the operation of the respiratory function measuring system 10 were explained. Then, the result is shown which was obtained from the respiratory function inspection of multiple subjects using the respiratory function measuring system 10 of the present embodiment. The first experiment to the fourth experiment is the experiments performed to healthy persons as subjects who did not have respiratory function difficulties, and the fifth experiment is the experiment performed to patients as subjects who had respiratory function difficulties. First, the first experiment to the third experiment show that the respiratory function measuring system 10 of the present embodiment has function equivalent to that of Spirometer. Further, the fourth experiment shows that the respiratory function measuring system 10 of the present embodiment has function equivalent to that of the respisomnogram. Furthermore, the fifth experiment shows the features of the respiratory waveforms of a patient who had a respiratory function difficulty, and the diagnostic method for deriving a medical view from the measurement result by this system.

### (Conditions of the experiment)

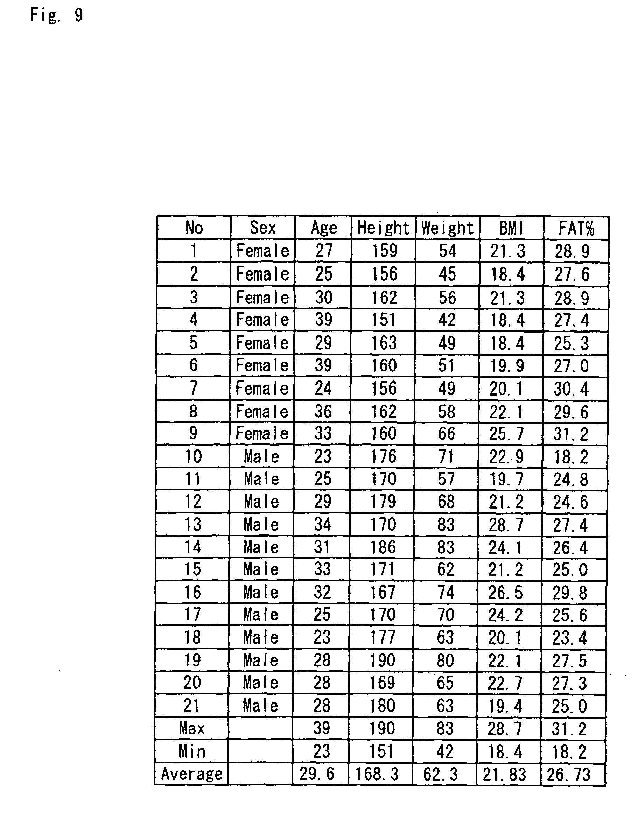

Respiratory function inspections by this system were conducted from July 15, 2000 to November 15, 2000 to 21 healthy persons (12 men and 9 women) who did not have respiratory function difficulties. At this time, the measurement by the Spirometer was also performed simultaneously with the measurement by this system, and both data were compared. The subject's sex, age, height, weight, BMI, and the rate of body fat are shown in Fig. 9. The size of the fixing unit 120 was 140 mm x 1170 mm, the sensing unit 110 was 125 mm x 230 mm, and the initial pressure was 30 mmHg for men and 20 mmHg for women. Since it was admitted by the applicant of the present invention that the measurement data when setting an initial pressurization value to 30 mmHg and the value to 20 mmHg could be equally treated without rectifying by preliminary experiment, the data obtained from men and women were treated equally and analyzed. And, the respiratory waveform data were generated with summing the chest data and the abdomen data.

### (Experiment 1) Measurement of lung volume fraction

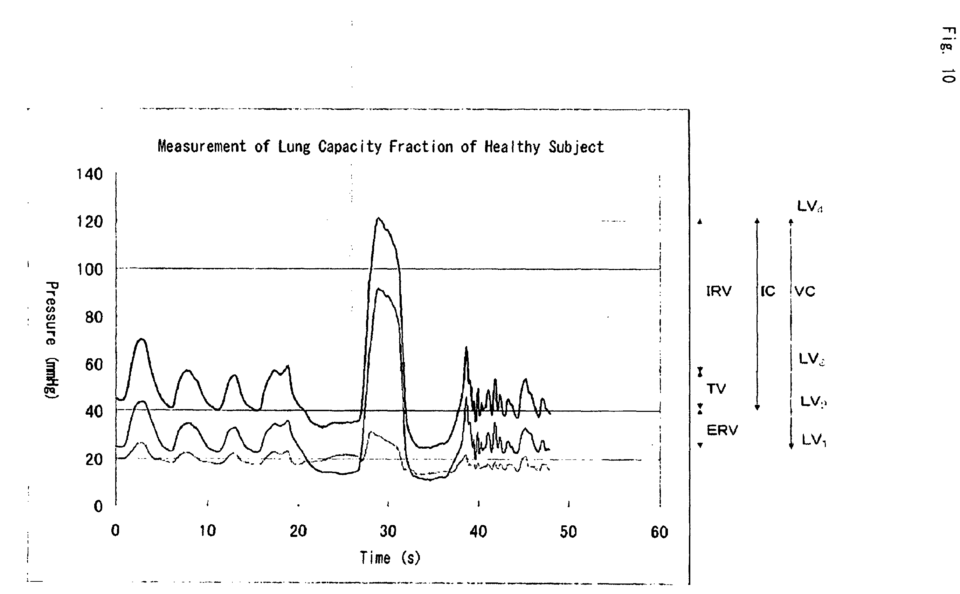

Fig. 10 shows change of inner pressure of the sensing unit 110 with passage of time when measuring the lung volume fraction of a subject. In the figure, a solid line shows the chest data which was measured by costal respiration measuring unit 100a, a thin solid line shows the abdomen data measured by abdominal respiration measuring unit 100b, and a thick solid line shows the respiratory waveform data which is the sum of each data. Moreover, LV1 shows the maximum expiratory level of the respiratory waveform data, LV2 shows the resting expiratory level of the respiratory waveform data, LV3 shows the resting inspiratory level of the respiratory waveform data, and LV4 shows the maximum inspiratory level of the respiratory waveform data.

### (Experiment 1-1) Measurement of the vital capacity VC

The vital capacity VC is given by the difference of the maximum expiratory level LV1 and the maximum inspiratory level LV4. Since inner pressure of the sensing unit 110 is not converted into the respiration volume in this experiment, the quantity computed from the measurement result shown in Fig. 10 is not the vital capacity VC itself, but it is the barometer VC1 for computing vital capacity. In the case of the subject 2, VC1 is calculated as 78.6 (mmHg) when only the chest data is used, and 12.42 (mmHg) when only the abdomen data are used, and 88.32 (mmHg) when the respiratory waveform data is used. The vital capacity VC was calculated as 2.68 (1) from a result measured simultaneously with the Spirometer.

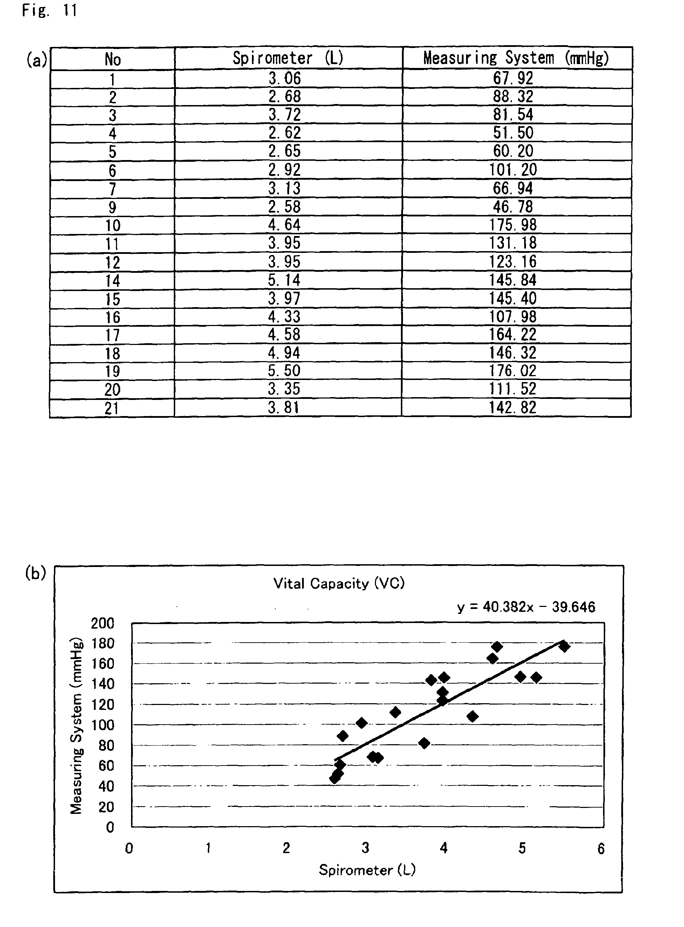

Similar measurement was performed to 21 subjects and the results were compared with the vital capacity VC measured by the Spirometer and evaluated. By the way, as for the subjects for whom the waveform of the measurement data disordered largely, the data were not used for evaluation. The vital capacity VC1 computed from each subject's respiratory waveform data is shown in Fig. 11 (A). Fig. 11(b) shows the measurement results VC1 measured by the respiratory function measuring system 10 plotted against the measurement results VC2 measured by the Spirometer. The least squares regression line is VC1 = 40.382 x VC2 - 39.646, and the correlation coefficient is 0.876 which is a high value.

### (Experiment 1-2) Measurement of the expiratory reserve volume ERV

The expiratory reserve volume ERV is given by the difference of the maximum expiratory level LV1 and the resting expiratory level LV2. Since inner pressure of the sensing unit 110 is not converted into the respiration volume in this experiment, the quantity computed from the measurement result shown in Fig. 10 is not the expiratory reserve volume ERV itself, but it is the barometer ERV1 for computing the expiratory reserve volume. In the case of the subject 2, ERV1 is calculated as 9.6 (mmHg) when only the chest data is used, and -1.12 (mmHg) when only the abdomen data are used, and 7.46 (mmHg) when the respiratory waveform data is used. The expiratory reserve volume ERV was calculated as 0.99 (1) from a result measured simultaneously with the Spirometer.

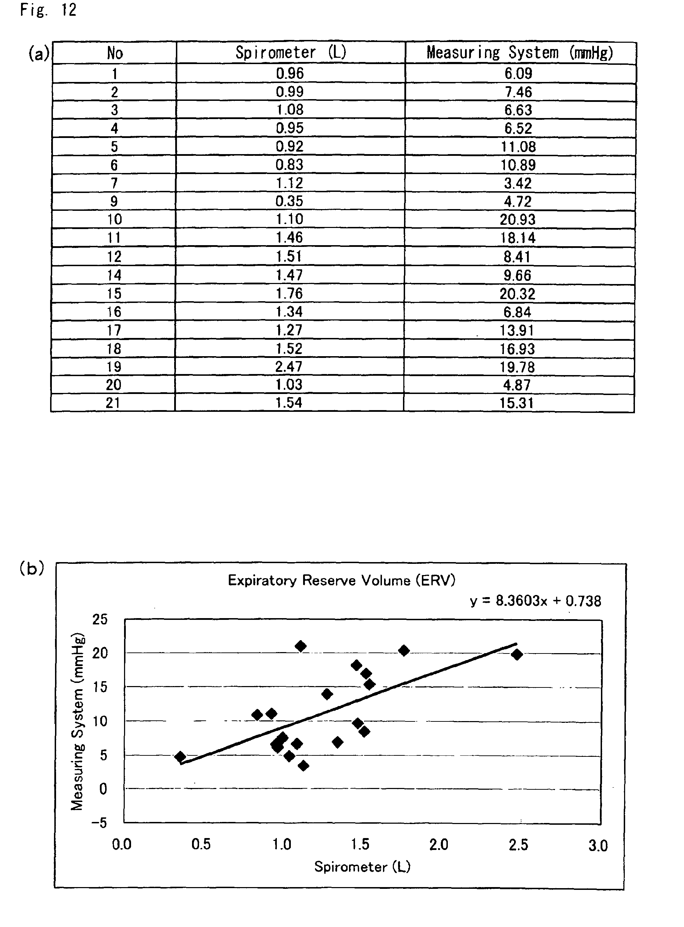

Similar measurement was performed to 21 subjects and the results were compared with the expiratory reserve volume ERV2 measured by the Spirometer and evaluated. By the way, as for the subjects for whom the waveform of the measurement data disordered largely, the data were not used for evaluation. The barometer of the expiratory reserve volume ERV1 computed from each subject's respiratory waveform data is shown in Fig. 12(a). Fig. 12(b) shows the measurement results ERV1 measured by the respiratory function measuring system 10 plotted against the measurement results ERV2 measured by the Spirometer. The least squares regression line is ERV1 = 8.3603 x ERV2 + 0.738, and the correlation coefficient is 0.635 which is a high value.

### (Experiment 1-3) Measurement of the inspiratory reserve volume IRV

The inspiratory reserve volume IRV is given by the difference of the maximum inspiratory level LV4 and the resting inspiratory level LV3. Since inner pressure of the sensing unit 110 is not converted into the respiration volume in this experiment, the quantity computed from the measurement result shown in Fig. 10 is not the inspiratory reserve volume IRV itself, but it is the barometer IRV1 for computing the inspiratory reserve volume. In the case of the subject 2, IRV1 is calculated as 57.33 (mmHg) when only the chest data is used, and 8.63 (mmHg) when only the abdomen data are used, and 64.25 (mmHg) when the respiratory waveform data is used. The inspiratory reserve volume IRV was calculated as 1.18 (1) from a result measured simultaneously with the Spirometer.

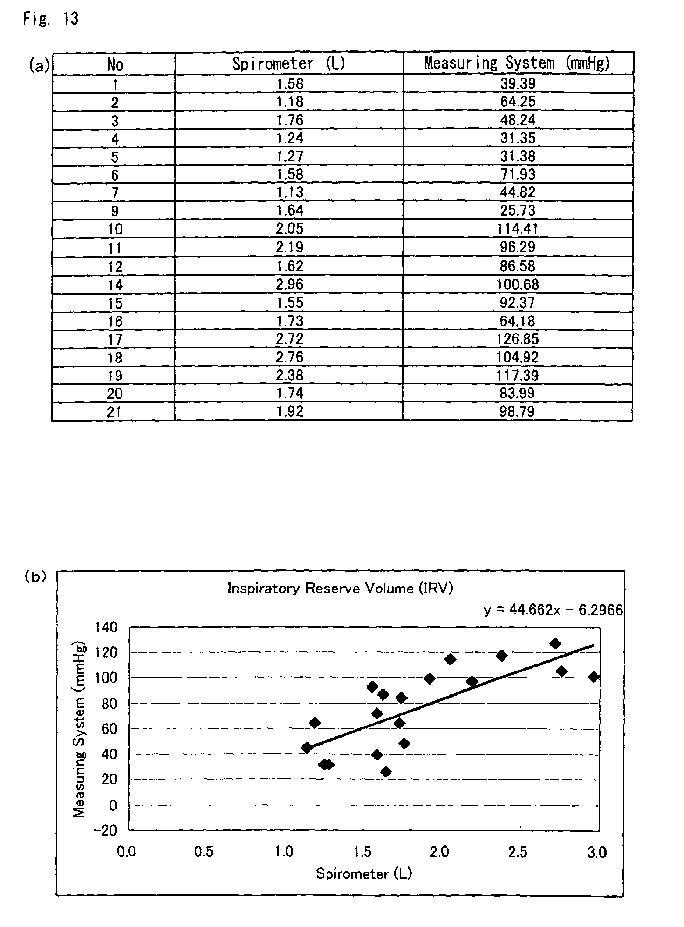

Similar measurement was performed to 21 subjects and the results were compared with the inspiratory reserve volume IRV2 measured by the Spirometer and evaluated. By the way, as for the subjects for whom the waveform of the measurement data disordered largely, the data were not used for evaluation. The barometer of the inspiratory reserve volume IRV1 computed from each subject's respiratory waveform data is shown in Fig. 13 (a) . Fig. 13(b) shows the measurement results IRV1 measured by the respiratory function measuring system 10 plotted against the measurement results IRV2 measured by the Spirometer. The least squares regression line is IRV1 = 44.662 x IRV2 - 6.2966, and the correlation coefficient is 0.756 which is a high value.

### (Experiment 1-4) Measurement of the inspiratory capacity IC

The inspiratory capacity IC is given by the difference of the maximum inspiratory level LV4 and the resting expiratory level LV2. Since inner pressure of the sensing unit 110 is not converted into the respiration volume in this experiment, the quantity computed from the measurement result shown in Fig. 10 is not the inspiratory capacity IC itself, but it is the barometer IC1 for computing the inspiratory capacity. In the case of the subject 2, IC1 is calculated as 69 (mmHg) when only the chest data is used, and 13.63 (mmHg) when only the abdomen data are used, and 80.86 (mmHg) when the respiratory waveform data is used. The inspiratory capacity IC was calculated as 1.69 (1) from a result measured simultaneously with the Spirometer.

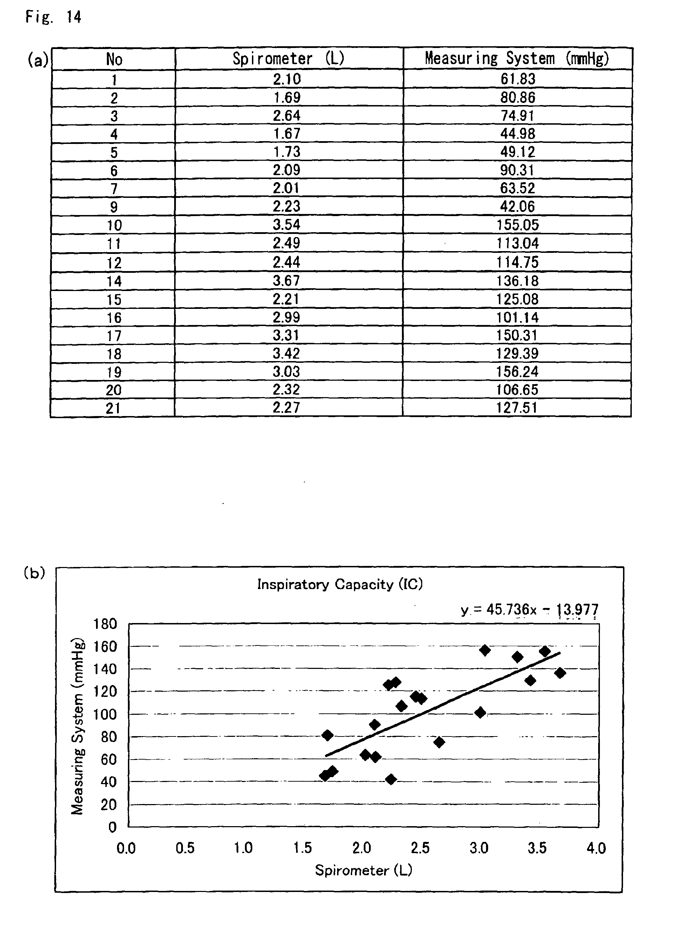

Similar measurement was performed to 21 subjects and the results were compared with the inspiratory capacity IC2 measured by the Spirometer and evaluated. By the way, as for the subjects for whom the waveform of the measurement data disordered largely, the data were not used for evaluation. The barometer of the inspiratory capacity IC1 computed from each subject's respiratory waveform data is shown in Fig. 14(a). Fig. 14(b) shows the measurement results IC1 measured by the respiratory function measuring system 10 plotted against the measurement results IC2 measured by the Spirometer. The least squares regression line is IC1 = 45.736 x IC2 - 13.977, and the correlation coefficient is 0.770 which is a high value.

### (Experiment 1-5) Measurement of the tidal volume TV

The tidal volume TV is given by the difference of the resting inspiratory level LV3 and the resting expiratory level LV2. Since inner pressure of the sensing unit 110 is not converted into the respiration volume in this experiment, the quantity computed from the measurement result shown in Fig. 10 is not the tidal volume TV itself, but it is the barometer TV1 for computing the tidal volume. In the case of the subject 2, TV1 is calculated as 11.67 (mmHg) when only the chest data is used, and 4.99 (mmHg) when only the abdomen data are used, and 16.61 (mmHg) when the respiratory waveform data is used. The tidal volume TV was calculated as 0.51 (1) from a result measured simultaneously with the Spirometer.

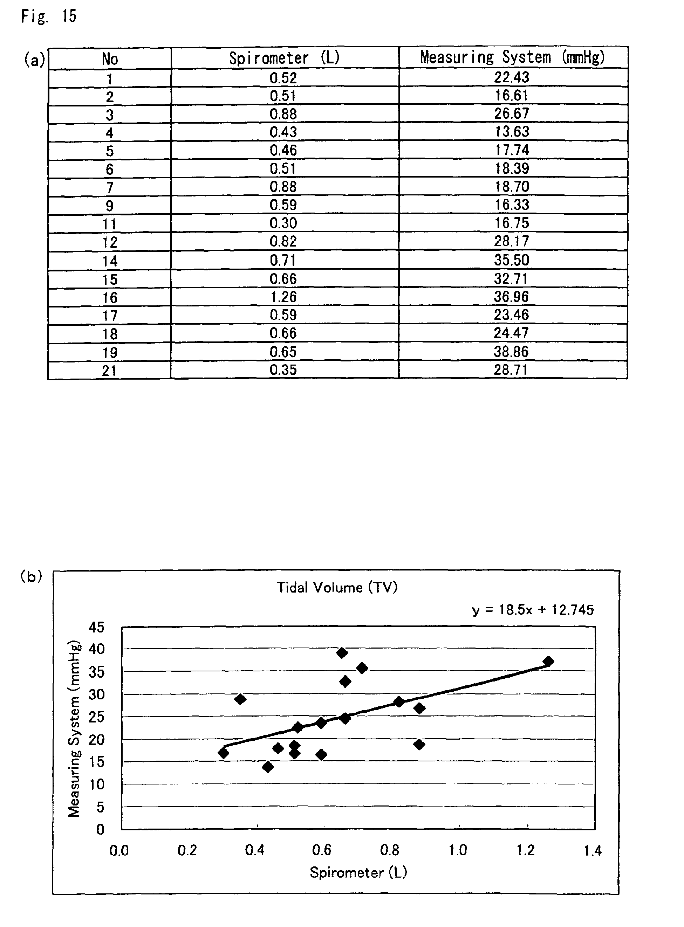

Similar measurement was performed to 21 subjects and the results were compared with the tidal volume TV2 measured by the Spirometer and evaluated. By the way, as for the subjects for whom the waveform of the measurement data disordered largely, the data were not used for evaluation. The barometer of the tidal volume TV1 computed from each subject's respiratory waveform data is shown in Fig. 15(a). Fig. 15(b) shows the measurement results TV1 measured by the respiratory function measuring system 10 plotted against the measurement results TV2 measured by the Spirometer. The least squares regression line is TV1 = 18.5 x TV2 + 12.745, and the correlation coefficient is 0.541 which is a high value.

From above results, it has been confirmed that the lung volume fraction can be measured by the respiratory function measuring system 10 as well as by the Spirometer. Moreover, the absolute value of the lung volume fraction can be obtained from the measurement data of this system using the formula of regression line. The conversion formula with high accuracy can be obtained by performing the same measurement to more subjects.

### (Experiment 2) Measurement of the forced expiratory curve

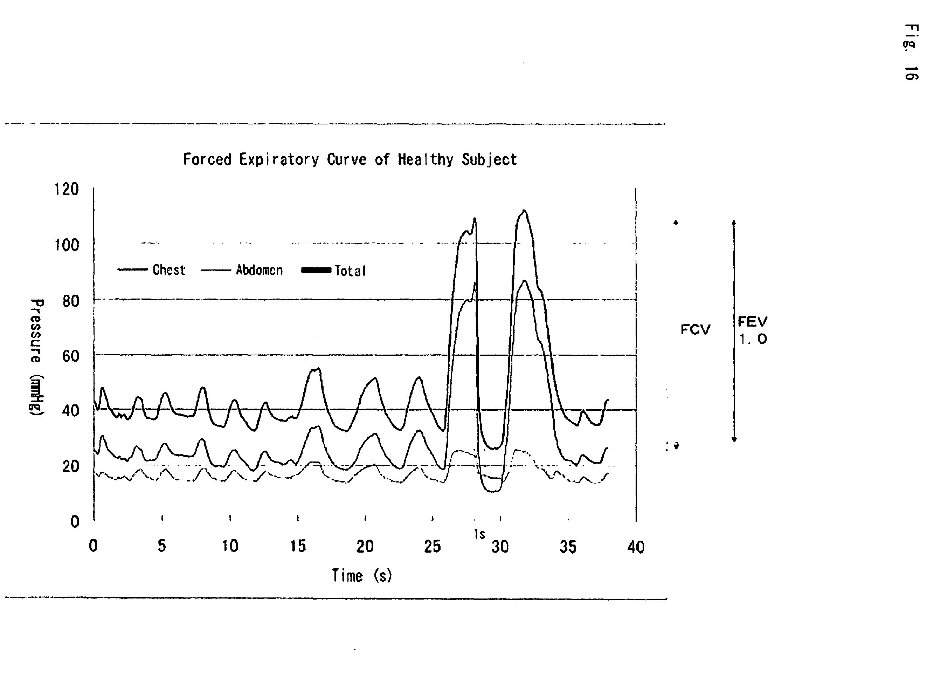

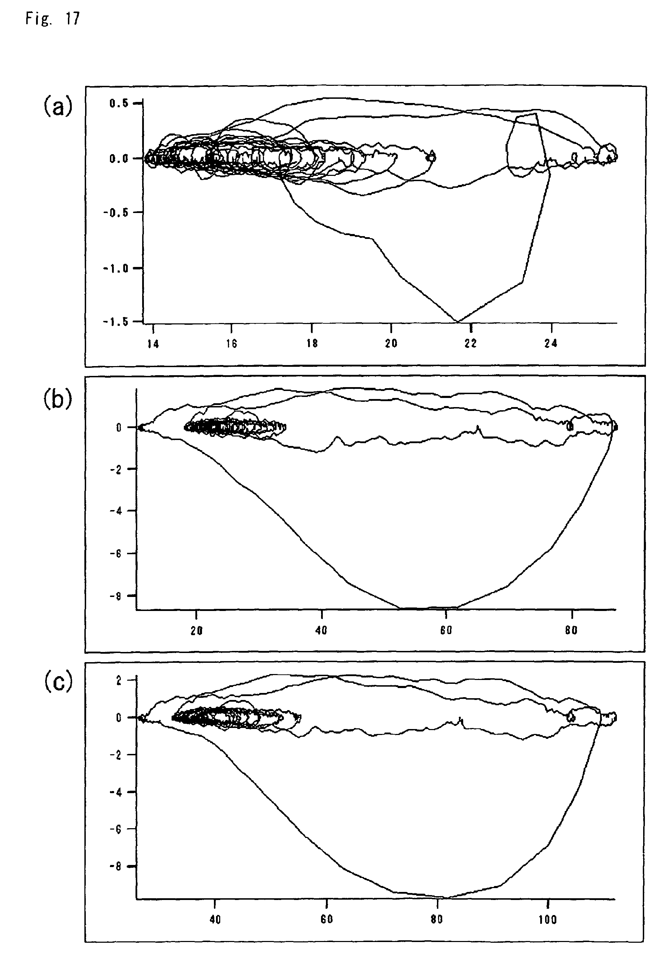

Fig. 16 shows change of inner pressure of the sensing unit 110 with time when measuring the forced expiratory curve of the subject 2. In the figure, a solid line shows the chest data measured by the costal respiration measuring unit 100a, a thin solid line shows the abdomen data measured by the abdominal respiration measuring unit 100b and a thick line shows the respiratory waveform data which is the sum of the each data. Fig. 17(a), 17(b), and 17(c) are the flow volume curves obtained by differentiating the forced expiration curve of Fig. 16. Fig. 17A shows the flow volume curve obtained from the abdomen data, Fig. 17(b) shows the flow volume curve obtained from the chest data, and Fig. 17(c) shows the flow volume curve obtained from the respiratory waveform data. Generally, the flow volume curve obtained by the Spirometer is represented with the respiratory volume on a horizontal axis and respiratory rate on a vertical axis, but in Fig. 17, the time on the horizontal axis. This is because the data was analyzed without converting them into the respiratory volume, but It is considered that there is no influence in calculation of the peak expiratory flow rate PEFR.

### (Experiment 2-1) Measurement of the forced vital capacity FVC

The forced vital capacity FVC is given by a method shown in Fig. 16. Since inner pressure of the sensing unit 110 is not converted into the respiration volume in this experiment, the quantity computed from the measurement result shown in Fig. 16 is not the forced vital capacity FVC itself, but it is the barometer FVC1 for computing the forced vital capacity. In the case of the subject 2, FVC1 is calculated as 75.8 (mmHg) when only the chest data is used, and 11.62 (mmHg) when only the abdomen data are used, and 83.32 (mmHg) when the respiratory waveform data is used. The forced vital capacity FVC was calculated as 2.57 (1) from a result measured simultaneously with the Spirometer.

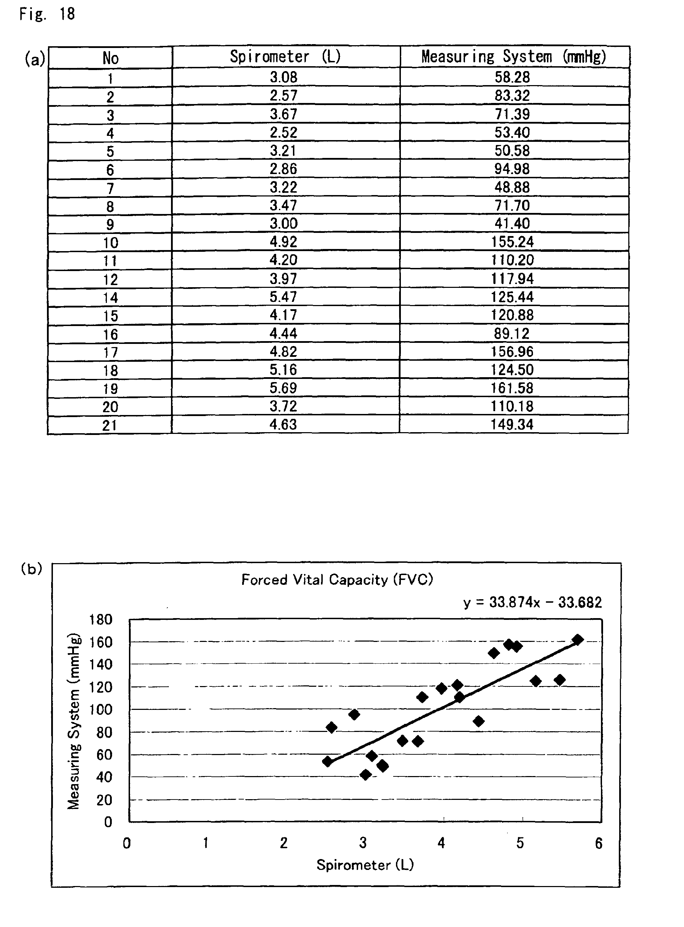

Similar measurement was performed to 21 subjects and the results were compared with the forced vital capacity FVC measured by the Spirometer and evaluated. By the way, as for the subjects for whom the waveform of the measurement data disordered largely, the data were not used for evaluation. The barometer of the forced vital capacity FVC1 computed from each subject's respiratory waveform data is shown in Fig. 18(a). Fig. 18(b) shows the measurement results FVC1 measured by the respiratory function measuring system 10 plotted against the measurement results FVC2 measured by the Spirometer. The least squares regression line is FVC1 = 33.874 x FVC2 - 33.682, and the correlation coefficient is 0.831 which is a high value.

### (Experiment 2-2) Measurement of the forced expiratory volume in one second FEV1.0

The forced expiratory volume in one second FEV1.0 is given by a method shown in Fig. 16. Since inner pressure of the sensing unit 110 is not converted into the respiration volume in this experiment, the quantity computed from the measurement result shown in Fig. 16 is not the forced expiratory volume in one second FEV1.0 itself, but it is the barometer FEV1.01 for computing the forced expiratory volume in one second. In the case of the subject 2, FEV1.01 is calculated as 75.6 (mmHg) when only the chest data is used, and 10.16 (mmHg) when only the abdomen data are used, and 82.84 (mmHg) when the respiratory waveform data is used. The forced expiratory volume in one second FEV1.0 was calculated as 2.51 (1) from a result measured simultaneously with the Spirometer.

Similar measurement was performed to 21 subjects and the results were compared with the forced expiratory volume in one second FEV1.0 measured by the Spirometer and evaluated. By the way, as for the subjects for whom the waveform of the measurement data disordered largely, the data were not used for evaluation. The barometer of the forced expiratory volume in one second FEV1.01 computed from each subject's respiratory waveform data is shown in Fig. 18(a). Fig. 18(b) shows the measurement results FEV1.01 measured by the respiratory function measuring system 10 plotted against the measurement results FEV1.02 measured by the Spirometer. The least squares regression line is FEV1.01 = 38.438 x FEV1.02-39.038, and the correlation coefficient is 0.790 which is a high value.

### (Experiment 2-3) Measurement of the peak expiratory flow rate PEFR

The peak expiratory flow rate PEFR is given by a method shown in Fig. 17. Since inner pressure of the sensing unit 110 is not converted into the respiration volume in this experiment, the quantity computed from the measurement result shown in Fig. 16 is not the peak expiratory flow rate PEFR itself, but it is the barometer PEFR1 for computing the peak expiratory flow rate. In the case of the subject 2, PEFR1 is calculated as 8.65 (mmHg/s) when only the chest data is used, and 1.51 (mmHg/s) when only the abdomen data are used, and 9.69 (mmHg/s) when the respiratory waveform data is used. The peak expiratory flow rate PEFR was calculated as 7 (1/s) from a result measured simultaneously with the Spirometer.

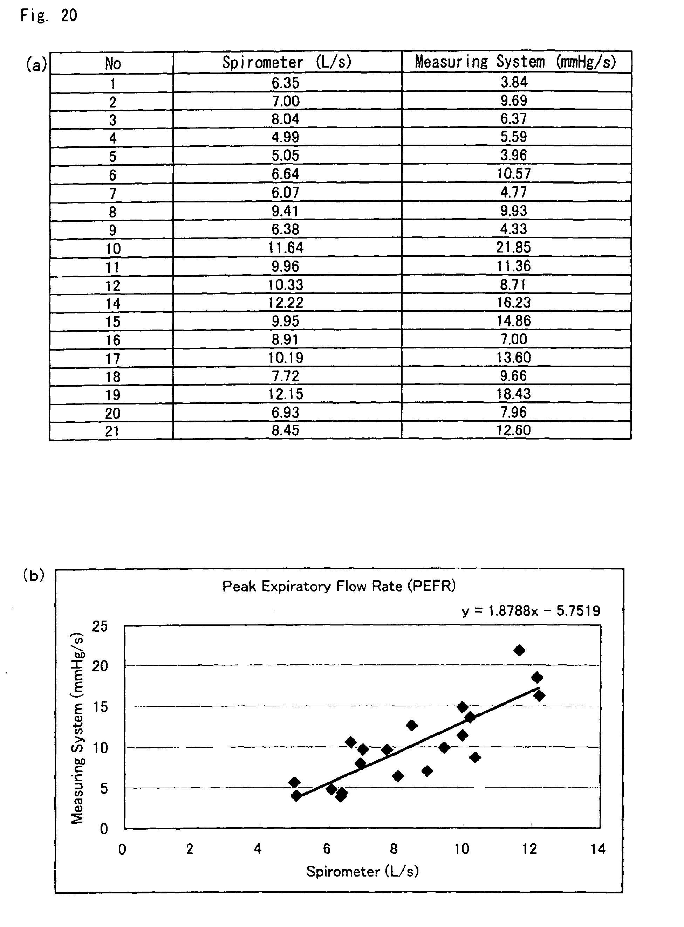

Similar measurement was performed to 21 subjects and the results were compared with the peak expiratory flow rate PEFR measured by the Spirometer and evaluated. By the way, as for the subjects for whom the waveform of the measurement data disordered largely, the data were not used for evaluation. The barometer of the peak expiratory flow rate PEFR1 computed from each subject's respiratory waveform data is shown in Fig. 20(a). Fig. 20(b) shows the measurement results PEFR1 measured by the respiratory function measuring system 10 plotted against the measurement results PEFR2 measured by the Spirometer. The least squares regression line is PEFR1 = 1.8788 x PEFR2 - 5.7519, and the correlation coefficient is 0.845 which is a high value.

From above results, it has been confirmed that the forced vital capacity, the forced expiratory volume in one second, the peak expiratory flow rate, the forced expiratory curve, and the flow volume curve, etc. can be measured by the respiratory function measuring system 10 as well as by the Spirometer. Moreover, the absolute values of above data can be obtained in the same way of the lung volume fraction from the measurement data of this system using the formula of the regression line. The conversion formula with high accuracy can be obtained by performing the same measurement to more subjects. (Experiment 3) Measurement of the resting ventilation volume

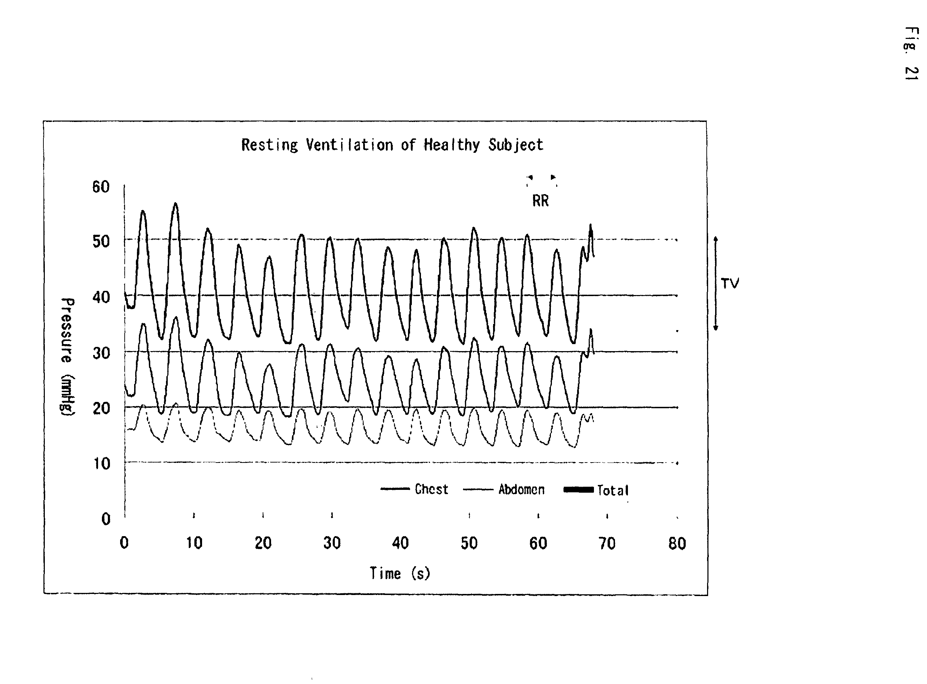

Fig. 21 shows change of inner pressure of the sensing unit 110 with time when measuring the resting ventilation volume of the subject 2. In the figure, a solid line shows the chest data measured by the costal respiration measuring unit 100a, a thin solid line shows the abdomen data measured by the abdominal respiration measuring unit 100b and a thick line shows the respiratory waveform data which is the sum of the each data.

### (Experiment 3-1) Measurement of the minutes volume MV

The minutes volume MV represents the ventilation volume of one minute, and is calculated by multiplying the one-time ventilation volume by the frequency of the respiration in one minute. Since inner pressure of the sensing unit 110 is not converted into the respiration volume in this experiment, the quantity computed from the measurement result shown in Fig. 21 is not the minutes volume MV itself, but it is the barometer MV1 for computing the minutes volume. In the case of the subject 2, MV1 is calculated as 175.34 (mmHg) when only the chest data is used, and 91.59 (mmHg) when only the abdomen data are used, and 278 (mmHg) when the respiratory waveform data is used. The minutes volume MV was calculated as 4.66 (1) from a result measured simultaneously with the Spirometer.

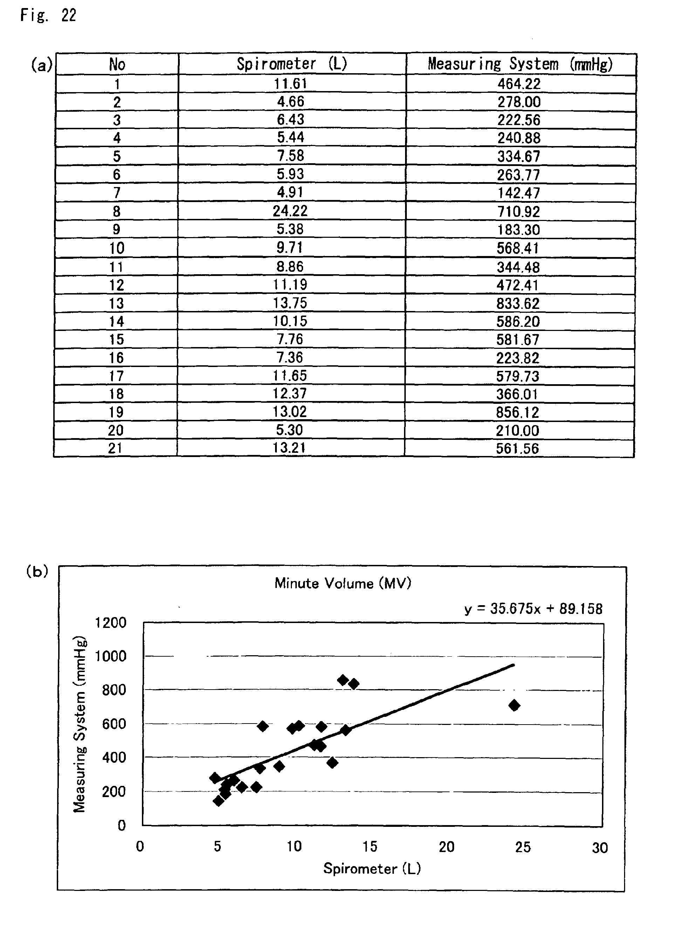

Similar measurement was performed to 21 subjects and the results were compared with the minutes volume MV measured by the Spirometer and evaluated. By the way, as for the subjects for whom the waveform of the measurement data disordered largely, the data were not used for evaluation. The barometer of the minutes volume MV1 computed from each subject's respiratory waveform data is shown in Fig. 22(a). Fig. 22(b) shows the measurement results MV1 measured by the respiratory function measuring system 10 plotted against the measurement results MV2 measured by the Spirometer. The least squares regression line is MV1 = 35.675 x MV2 + 89.158, and the correlation coefficient is 0.755 which is a high value.

### (Experiment 3-2) Measurement of the tidal volume TV

The tidal volume TV is calculated by averaging the differences between the peaks and the valleys of the respiratory waveform at resting. Since inner pressure of the sensing unit 110 is not converted into the respiration volume in this experiment, the quantity computed from the measurement result shown in Fig. 21 is not the tidal volume TV itself, but it is the barometer TV1 for computing the tidal volume. In the case of the subject 2, TV1 is calculated as 11.99 (mmHg) when only the chest data is used, and 6.25 (mmHg) when only the abdomen data are used, and 18.18 (mmHg) when the respiratory waveform data is used. The tidal volume TV was calculated as 0.33 (1) from a result measured simultaneously with the Spirometer.

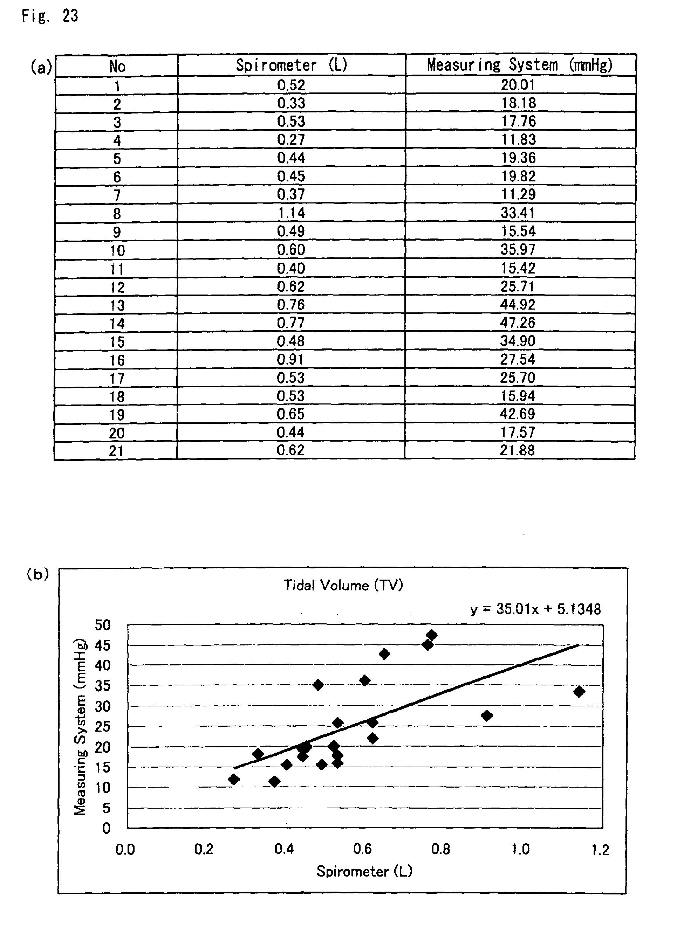

Similar measurement was performed to 21 subjects and the results were compared with the tidal volume TV measured by the Spirometer and evaluated. By the way, as for the subjects for whom the waveform of the measurement data disordered largely, the data were not used for evaluation. The barometer of the tidal volume TV1 computed from each subject's respiratory waveform data is shown in Fig. 23(a). Fig. 23(b) shows the measurement results TV1 measured by the respiratory function measuring system 10 plotted against the measurement results TV2 measured by the Spirometer. The least squares regression line is TV1 = 35.01 x TV2 + 5.1348, and the correlation coefficient is 0.649 which is a high value.

### (Experiment 3-3) Measurement of the interval of the waveform RR

The interval of the waveform RR is calculated by averaging the intervals between the peaks of the respiratory waveform at resting but in this experiment the reciprocal thereof is calculated as the frequency of the respiration per one minute. In the case of the subject 2, RR1 is calculated as 13.97 (/min) when only the chest data is used, and 13.98 (/min) when only the abdomen data are used, and 14 (/min) when the respiratory waveform data is used. The interval of the waveform RR was calculated as 14.12 (/min) from a result measured simultaneously with the Spirometer.

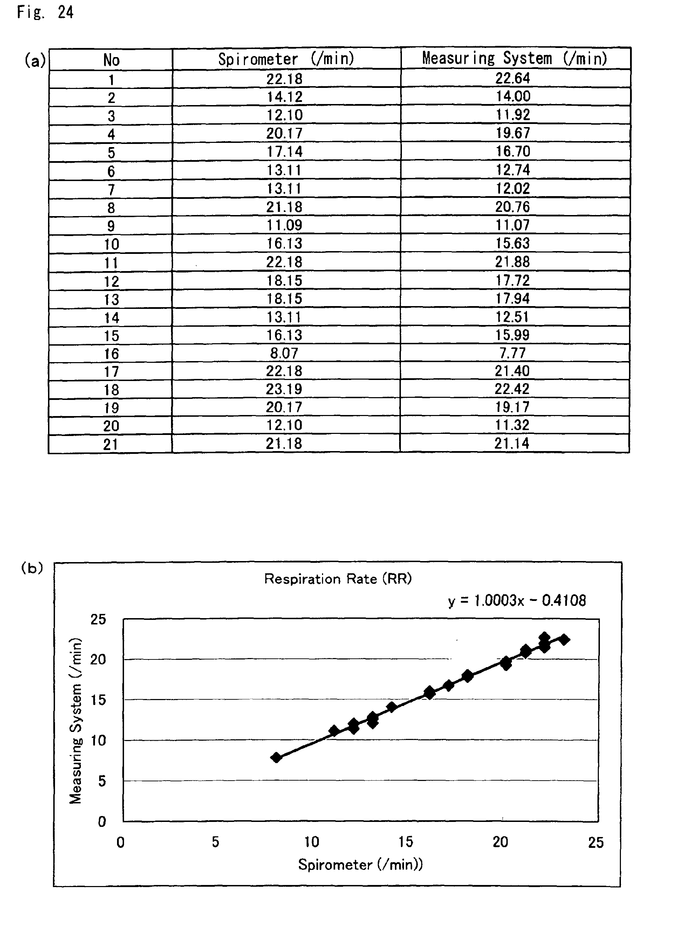

Similar measurement was performed to 21 subjects and the results were compared with the interval of the waveform RR measured by the Spirometer and evaluated. By the way, as for the subjects for whom the waveform of the measurement data disordered largely, the data were not used for evaluation. The barometer of the interval of the waveform RR1 computed from each subject's respiratory waveform data is shown in Fig. 24(a). Fig. 24(b) shows the measurement results RR1 measured by the respiratory function measuring system 10 plotted against the measurement results RR2 measured by the Spirometer. The least squares regression line is RR1 = 1.0003 x RR2 - 0.4108, and the correlation coefficient is 0.997 which is a high value.

From above results, it has been confirmed that the resting ventilation minute volume, the tidal volume, and the rate of the respiratory waveform etc. can be measured by the respiratory function measuring system 10 as well as by the Spirometer. Moreover, the absolute values of above data can be obtained in the same way of the lung volume fraction from the measurement data of this system using the formula of the regression line. The conversion formula with high accuracy can be obtained by performing the same measurement to more subjects.

As mentioned above, it has been confirmed that the data equivalent to those by Spirometer were obtained by this system. However, the advantage of this system does not remain in it, but this system has another big advantage that the chest data and the abdomen data can be measured independently. Below, the relation between the chest data and the abdomen data is explained.

### (Experiment 4) Relation between the chest data and the abdomen data

The relation of the chest data and abdomen data in the above-mentioned experiment was evaluated. Below, the measurement data at a time of resting and the measurement data at a time of being forced expiration are shown. (Experiment 4-1) Relation between the chest data and the abdomen data at a time of resting

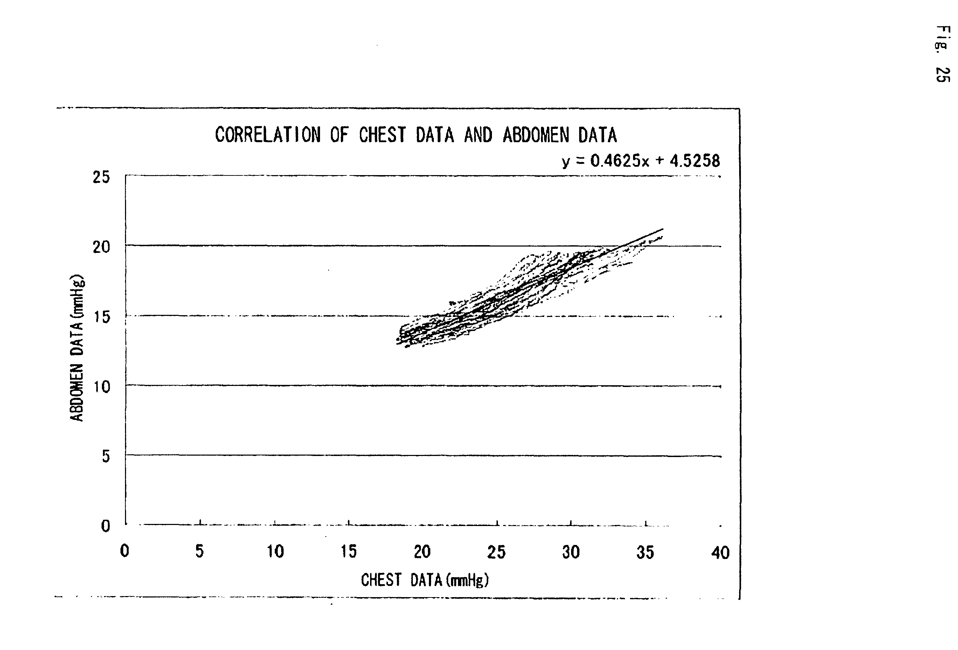

Fig. 25 shows the relation between the chest data and the abdomen data when the resting ventilation volume was measured in experiment 3. In Fig. 25, the chest data is expressed on the horizontal axis and the abdomen data is expressed on the vertical axis. Regressing them in a straight line by the least-squares method, (abdomen data) = 0.4625 x (chest data) + 4.5258, and the correlation coefficient showed 0.940 which is a high numerical value. This means that the subject had little variation in breathing at a time of resting, and that the costal respiration and the abdominal respiration were performed in an approximately same ratio.

### (Experiment 4-2) Relation between the chest data and the abdomen data at a time of being forced expiration

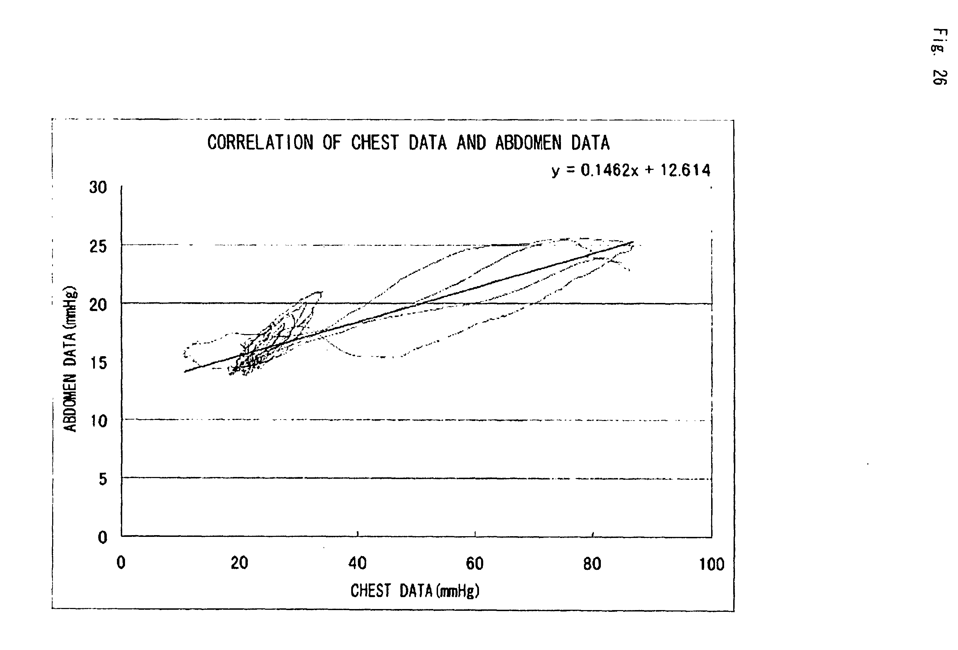

Fig. 26 shows the relation of the chest data and the abdomen data when the forced expiration curve was measured in experiment 2. In Fig. 26, the chest data is expressed on the horizontal axis and the abdomen data is expressed on the vertical axis. Regressing them in a straight line by the least-squares method, (abdomen data) = 0.1462 x (chest data) + 12.614, and the correlation coefficient was 0.871. Since the gradient of the regressing straight line is decreasing compared with that at a time of resting, it is found that the predominancy of the costal respiration at a time of being forced is stronger than that at a time of resting.

As mentioned above, it has been confirmed that the measuring system 10 of this embodiment can measure the chest data and the abdomen data independently, and the respiration predominancy and the rate of abdomen contribution can be evaluated as well as the respisomnogram. Furthermore, although the absolute value of the data obtained by the respisomnogram was not reliable and the quantitative discussion can not be done, in this system the reliable data can be obtained quantitatively as shown by explanation of experiments 1-3. Thereby, comparison examination can be quantitatively performed regarding multiple measurements of the same subject and multiple subjects' measurement.

As mentioned above, it has been shown that functions of the measurement system 10 of this embodiment are equivalent to those of the Spirometer and the respisomnogram by the experiments 1-4. The measurement system 10 of this embodiment has not only the functions of the both apparatus of the Spirometer and the respisomnogram, but also the measurement system 10 of this embodiment can obtain the information with one measurement which had to be conventionally acquired separately in the case of the spirometer and the respisomnogram. Moreover, this measurement system can provide a medical view by measuring the chest data and the abdomen data independently, which is not possible with the conventional the Spirometer and the respisomnogram.

For example, in the case of a patient whose upper leaf of lung was excised because of a disease such as emphysema, the respiratory muscles in the breast hardly contribute to breathing, and the patient almost breathes by the abdominal respiration, but the spirometer cannot acquire this view. Although it can be known that the abdominal respiration is predominant by the respisomnogram, the quantitative evaluation is difficult to be made since it lacks in reproducibility. However, since the measurement system 10 of this embodiment can acquire the data of costal respiration and abdominal respiration independently with sufficient reproducibility, progresses after an operation and/or the effect of rehabilitation or medicine etc can be observed and evaluated quantitatively, which is extremely meaningful medically.

Moreover, for an ALS patient (Amyotrophic Lateral Sclerosis) the degree of advancement of paralysis of the respiratory muscle which contributes to the costal respiration is observable by the measurement system 10 of this embodiment. In this case, the measurement system of this embodiment can also perform quantitative evaluation with higher reproducibility and higher reliability compared with the conventional respisomnogram.

### (Experiment 5) Measurement of patients as subjects who had respiratory disease

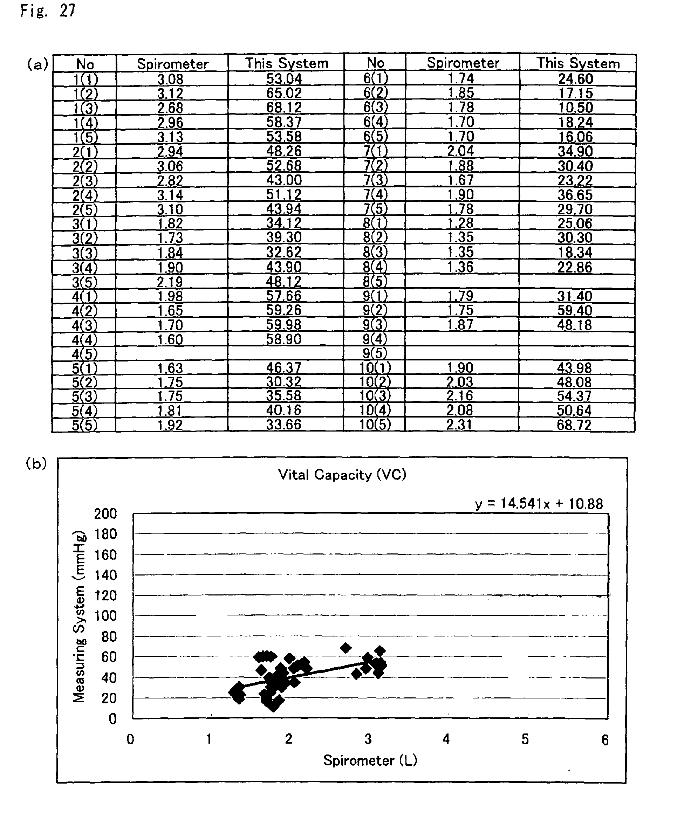

The respiratory function measurement by this system and the spirometer was performed similar to experiments 1-4 with making respiratory disease patients as subjects. The measurement was performed five times for each of 10 respiratory disease patients, respectively. As for the subjects for whom the waveform of the measurement data disordered largely, the data were not used for evaluation. The measurement results shown in Fig. 27 to 36 were analyzed like experiments 1-3 using the total value of the chest data and the abdomen data. The graph which describes the correlation with the spirometer is shown in the same scale with those of the experiments 1-3 for which the healthy persons were subjects.

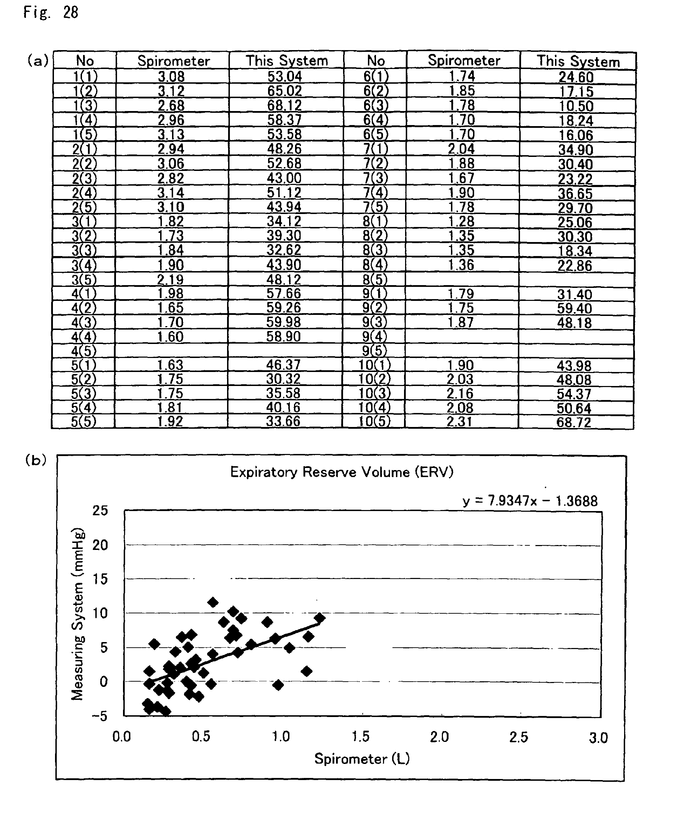

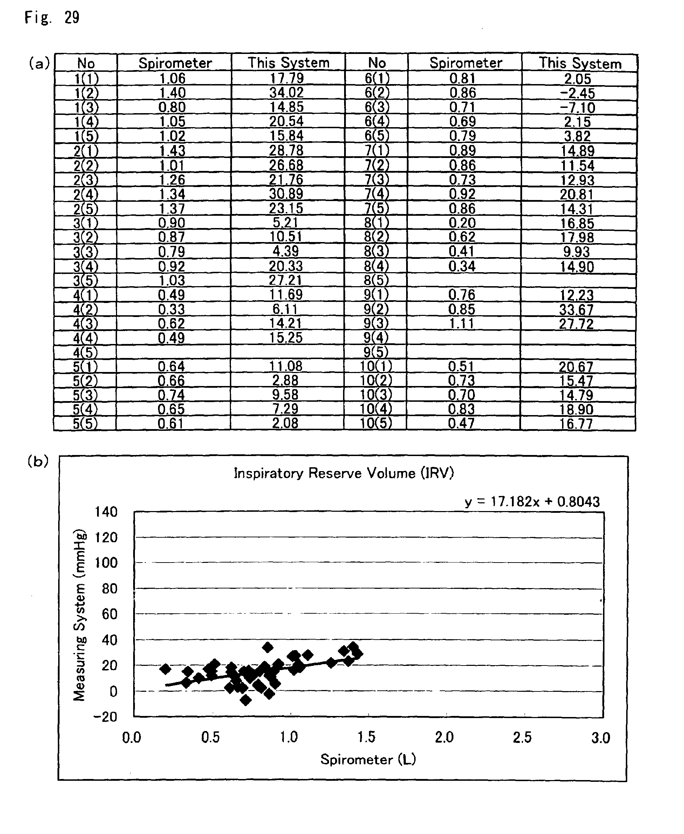

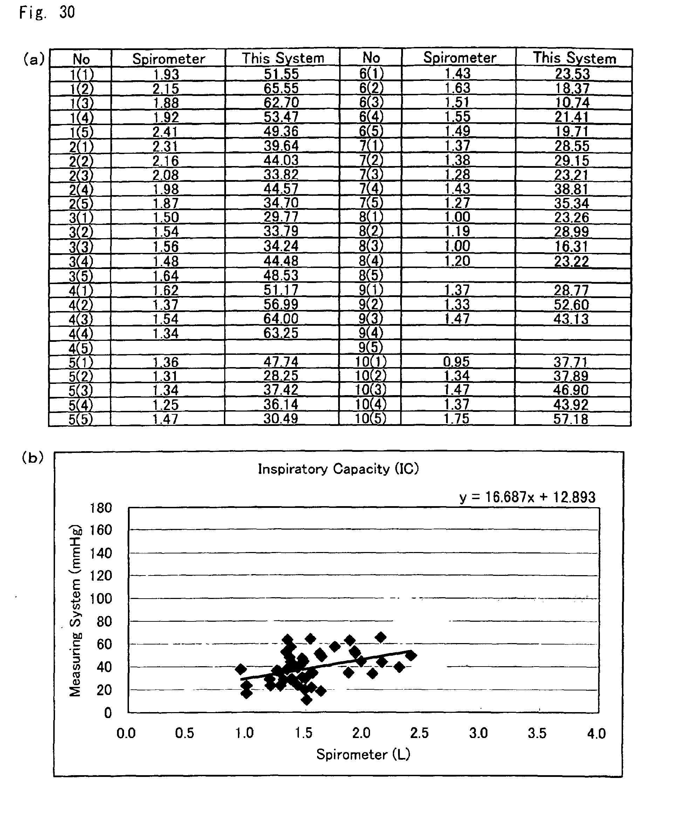

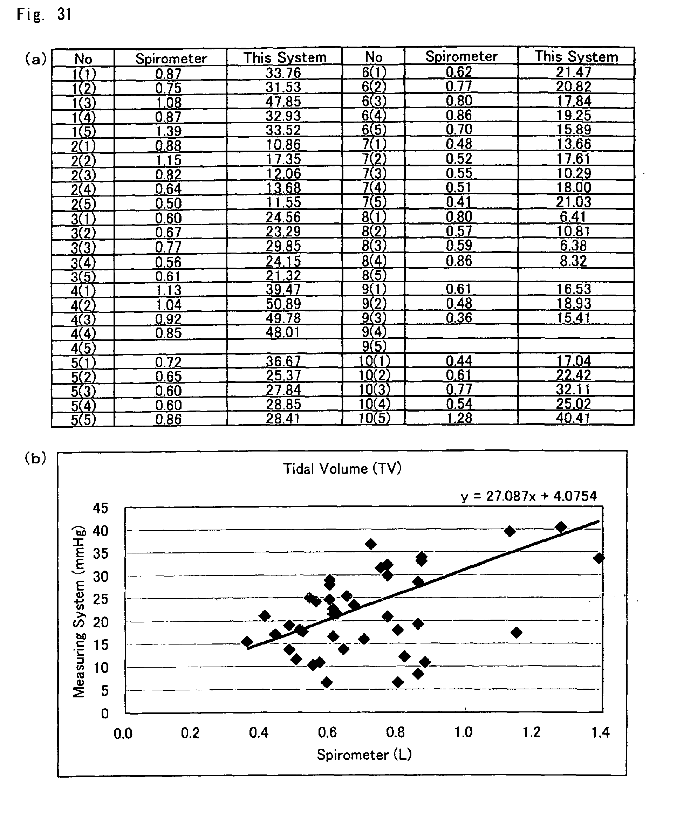

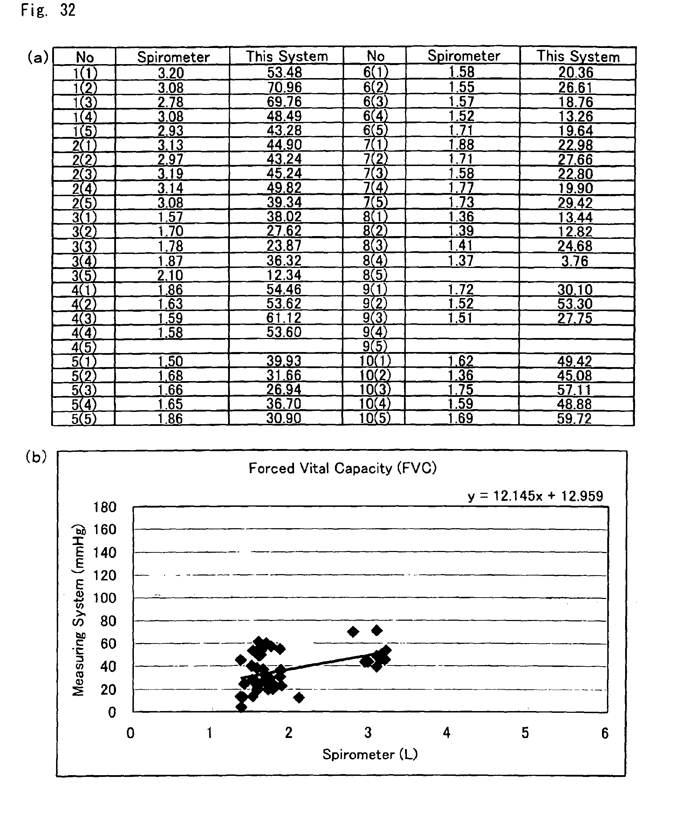

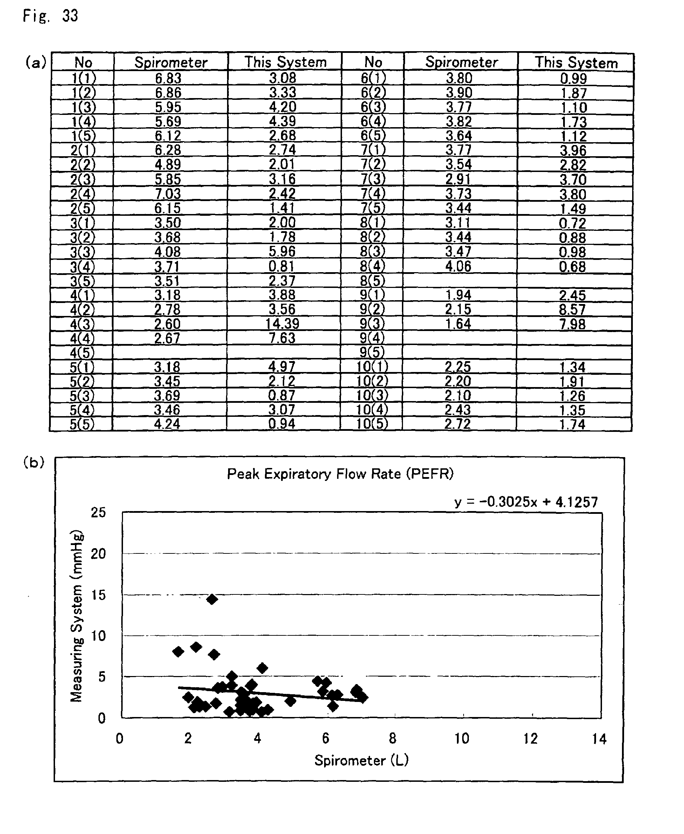

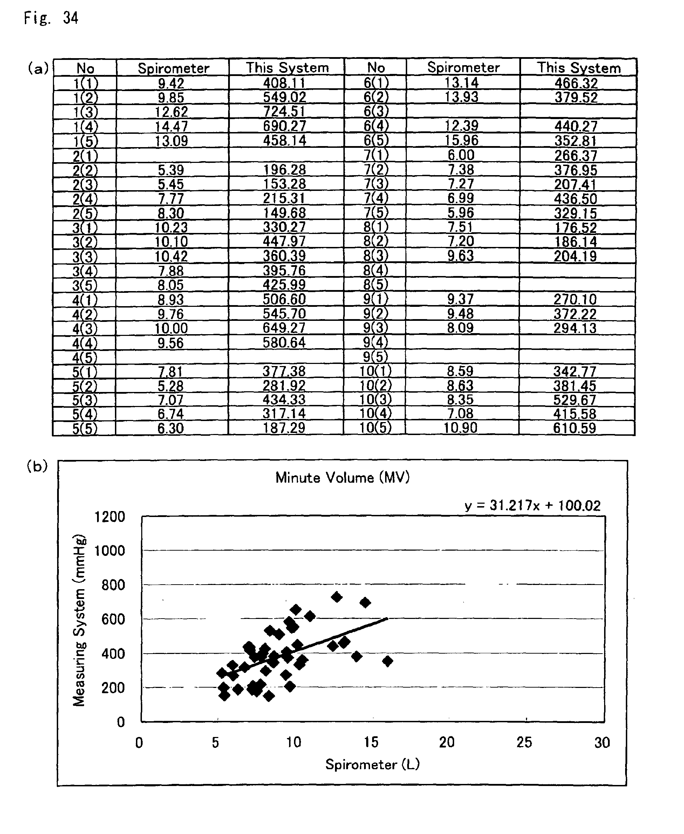

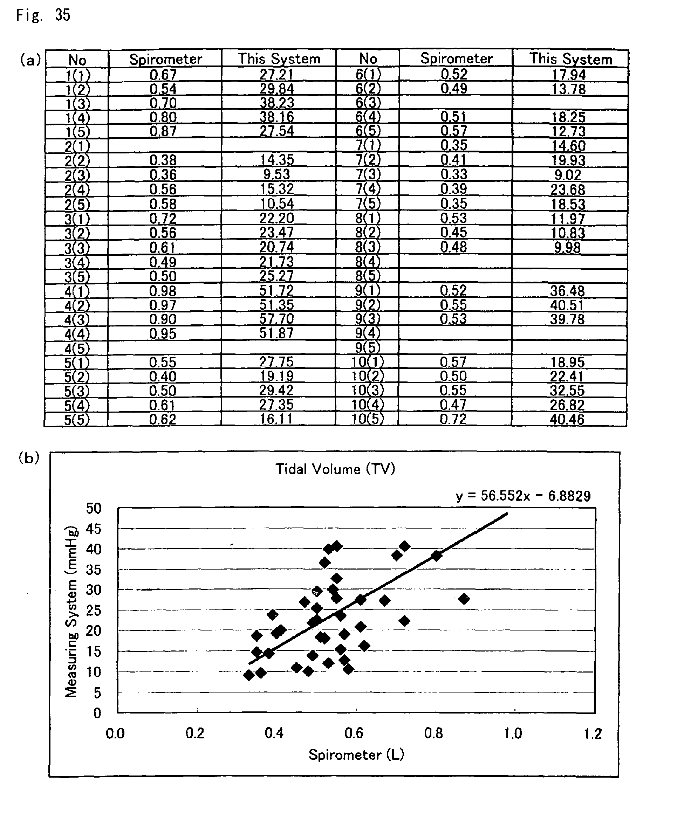

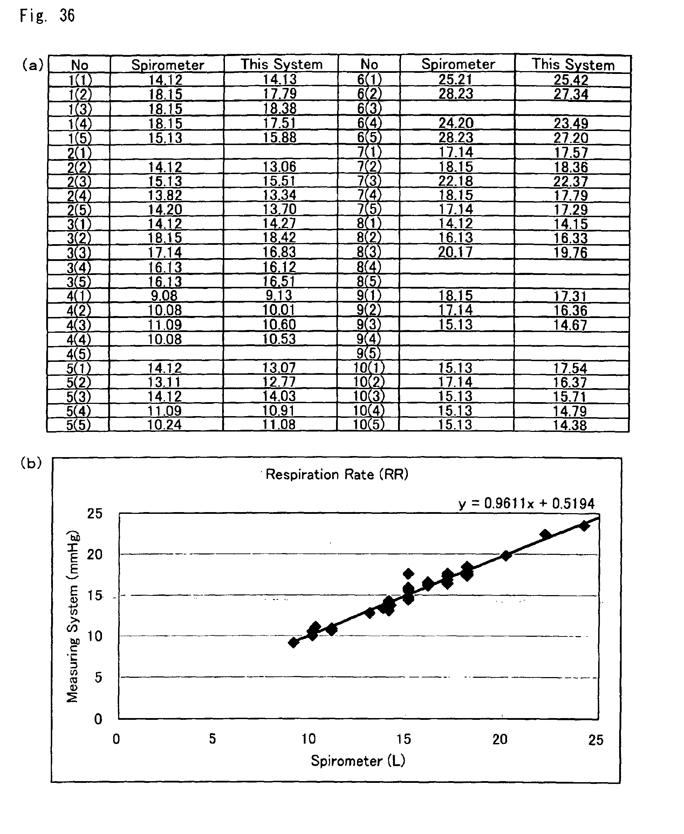

Fig. 27(a) shows the measurement result of the vital capacity VC, and Fig. 27(b) shows the correlation with data by spirometer. Fig. 28(a) shows the measurement result of the expiratory reserve volume ERV, and Fig. 28(b) shows the correlation with data by spirometer. Fig. 29(a) shows the measurement result of the inspiratory reserve volume IRV, and Fig. 29(b) shows the correlation with data by spirometer. Fig. 30(a) shows the measurement result the inspiratory capacity IC, and Fig. 30(b) shows the correlation with data by spirometer. Fig. 31(a) shows the measurement result of tidal volume TV, and Fig. 31(b) shows the correlation with data by spirometer. Fig. 32(a) shows the measurement result of the forced expiratory volume FVC, and Fig. 32(b) shows the correlation with data by spirometer. Fig. 33(a) shows the measurement result of the peak expiratory flow rate PEFR, and Fig. 33(b) shows the correlation with data by spirometer. Fig. 34(a) shows the measurement result of the resting ventilation minute volume MV, and Fig. 34(b) shows the correlation with data by spirometer. Fig. 35(a) shows the measurement result of tidal volume TV, and Fig. 35(b) shows the correlation with data by spirometer. Fig. 36(a) shows the measurement result of the respiratory rate RR, and Fig. 36(b) shows the correlation with data by spirometer.

From the above measurement results, it is shown that various kinds of respiratory function measurement can be performed by the measurement system of this embodiment also to respiratory disease patients. Moreover, we performed multiple measurements for each subject, and have confirmed that it was possible to obtain the highly reproducible measurement results.

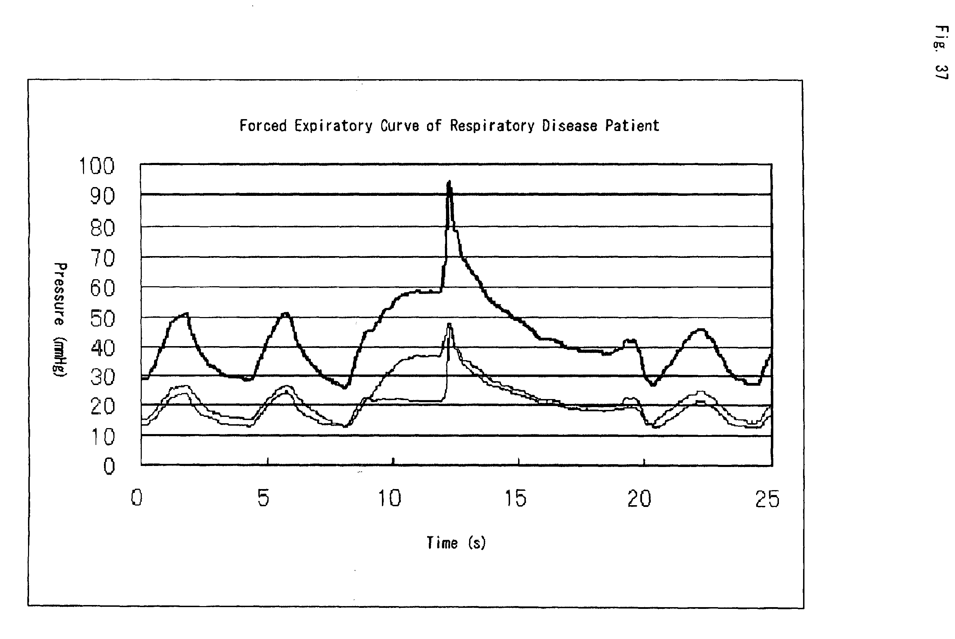

Fig. 37 shows change of inner pressure inside the sensing unit 110 with passage of time when measuring a certain respiratory disease patient's forced expiration curve. The solid line shows the chest data measured by costal respiratory measuring unit 100a, the thin solid line shows the abdomen data measured by abdominal respiratory measuring unit 100b, and the thick solid line shows the respiratory waveform data which is the sum of the chest data and the abdomen data. It is shown that the inner pressure went up rapidly at the moment of the forced expiration after the subject performed a maximum inspiration. This rapid rise of inner pressure is considered for the reason of constriction of the respiratory tract because; it was observed at the time of measurement of the forced expiration curve, it was often observed for asthmatic patients, but hardly observed for patients of bronchiectasis, and the pressure rises rapidly at the moment of a forced expiration, etc. Namely, it is considered that the inner pressure of the sensing unit 110 went up, since the air current was restricted in the constriction area of the respiratory tract or the respiratory muscle worked differently than that of a healthy person, when the subject tried to exhale at a blast right after inhalation.

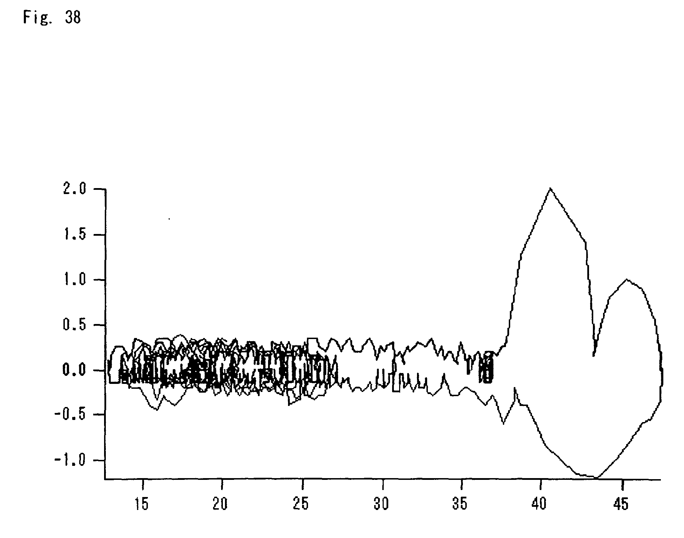

Fig. 38 shows the flow volume curve of a certain respiratory disease patient as a subject. Unlike the flow volume curve of a healthy person shown in Fig. 17, a big peak is seen in the positive direction. This is considered to reflect the rapid rise of inner pressure at the time of forced expiration mentioned above.

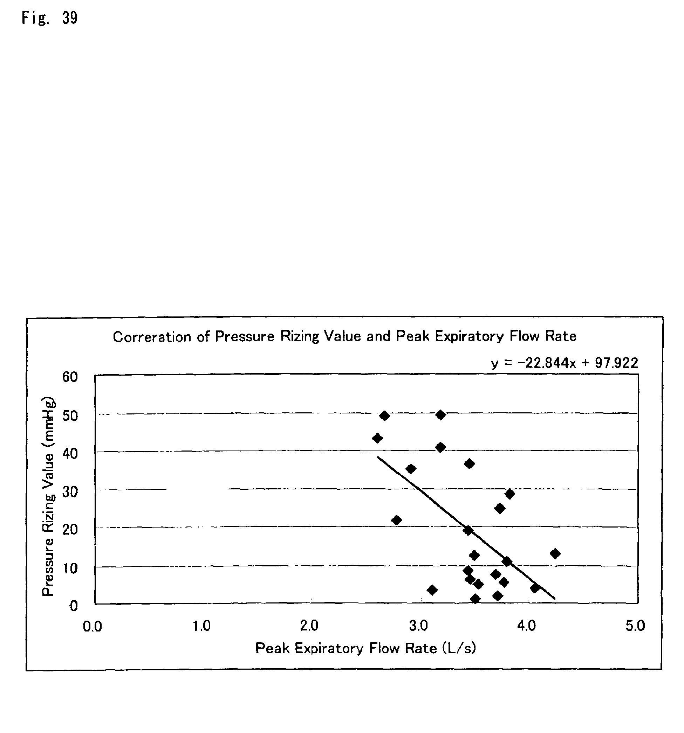

Fig. 39 is a figure showing the relation between the inner pressure rising value at the time of forced expiration, and the peak expiration flow rate PEFR. The inner pressure rise value at the time of forced expiration, which was observed when the forced expiration curve was measured for respiratory disease patients as subjects, shows a negative correlation, and it is indicated that the rise of pressure is reflecting the degree of constriction or blockage of a respiratory tract. From the above measurement results, it is shown that this system is effective to diagnose the state of constriction or blockage of a respiratory tract.

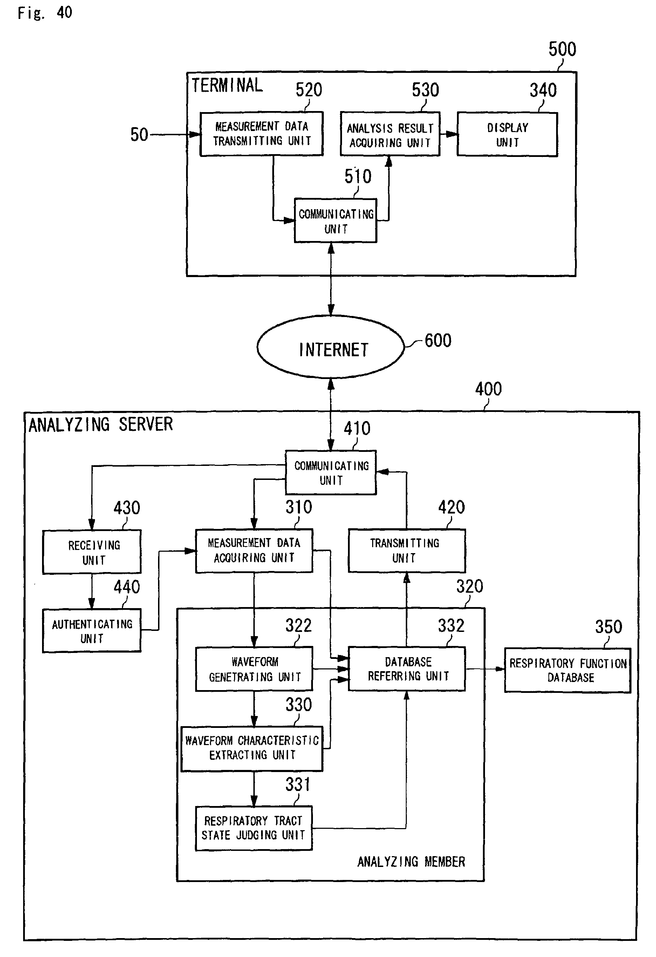

Fig. 40 shows a structure of an analyzing server 400 and a terminal 500 which accesses the analyzing server 400 according to this embodiment. The analyzing server 400 receives an analysis request from the terminal 500 which is installed in the hospital for example, through the Internet 600, and refers it to the respiratory function database 350 based on the received measurement data, and then transmits the analysis result to the terminal 500. The Internet 600 is mentioned as an example of the network in this embodiment , but it may be the networks of a cable or radio such as a cellular-phone network, a public network, etc.

The analyzing server 400 includes a communicating unit 410 for communicating with the terminal 500 through the Internet 600, a receiving unit 430 which receives the request for referring to the database through the network, an authenticating unit 440 which authenticates a user who refers to the database, the measurement data acquiring unit 310, the analyzing member 320, the respiratory function database 350, and a transmitting unit 420 for transmitting analysis results such as a medical view to the terminal 500. The same mark is given to what has a function equivalent to the composition shown in Fig. 1 among the composition of the analyzing server 400.

The analyzing server 400 first authenticates whether the user is a permitted user for the reference of the database by the authenticating unit 440 when a reference request of the database is received by the receiving unit 430 through the Internet 600. Then when the user is authenticated, the measurement data acquiring unit 310 receives the chest data and the abdomen data like the analyzing unit 300 shown in Fig. 1, and the respiratory waveform data is generated by the waveform generating unit 322, the waveform characteristics of the chest data, the abdomen data, and the respiratory waveform data is extracted by the extracting unit 330, and the constriction state of a respiratory tract is judged by the respiratory tract state judging part 331, and then the medical view is acquired by referring the respiratory function database 350 by the database referring unit 332. The acquired medical view is transmitted to a user through the Internet 600 from the transmitting unit 420. Further, an accounting unit may be attached to charge the user who refers to the database.

The analyzing server 400 shown in Fig. 40 provides mainly the reference service with managing the respiratory function database 350, and the composition such as the lung capacity fraction calculating unit 324 etc. is omitted, but of course the analyzing server 400 may include these compositions. The composition such as the lung capacity calculating unit 324 may be added to the terminal 500 side. In this case, the measurement data acquiring unit 310 may acquire the respiratory function barometers, such as the lung capacity fraction, from the terminal 500.

The terminal 500 includes a measurement data transmitting unit 520 for transmitting data which the measuring apparatus 50 measures to the analyzing server 400, a communicating unit 510 for communicating with the analyzing server 400 through the internet 600, an analysis result acquiring unit 530 for acquiring the analysis result from the analyzing server 400, and the display unit 340 which displays the analysis result at the display apparatus.

Thus, a service for analyzing the respiratory function can be provided to more users by providing a function equivalent to the analyzing unit 300 in Fig. 1 as the server. Moreover, the maintenance such as a renewal and/or modification of the database can be made easily by managing the respiratory function database 350 at the analyzing server 400 in the block without placing them at each terminal 500.

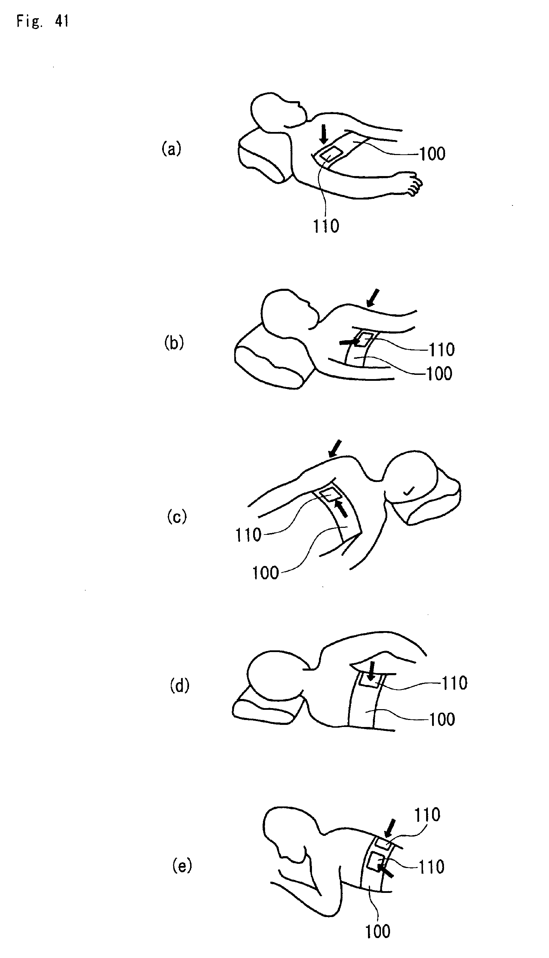

Fig. 41(a), 41(b), 41(c), 41(d), and 41(e) show examples how to use a rehabilitation assisting apparatus according to the embodiment. The rehabilitation assisting apparatus has the same composition as the measuring apparatus 50 shown in Fig. 1, and the measuring unit 100 is used as a pressurizing unit and the sensing unit 110 is used as the pressurizing member.

Fig. 41(a) shows how the upper leaf of the subject's lung is squeezed with the pressurizing unit 100. At this time, the pressurizing unit 110 is fixed in the area above the 4th rib, and gas is sent inside the pressurizing unit 110 to become the predetermined pressure by the pressurizing pump, so that the expectoration part is squeezed. Fig. 41(b) and 41(c) show how the middle part of a subject's lung is squeezed with the pressurizing unit 100. The right side of the lung is squeezed in Fig. 41(b) and the right side in Fig 41(c). Fig. 41(d) shows how the lower leaf of a subject's lung is squeezed with the pressurizing unit 100. Fig. 41(e) shows how the rear bottom side of a subject's lung is squeezed with the pressurizing unit 100. To squeeze appropriate part, multiple pressurizing units 100 may be equipped, and/or the pressurizing unit 100 may be attached with multiple pressurizing parts 110, if needed.

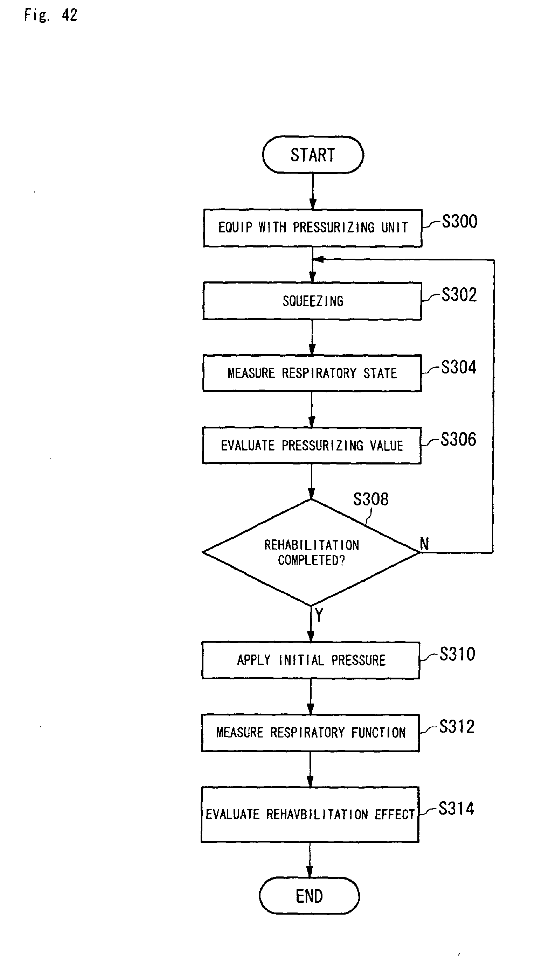

Fig. 42 shows the procedure of a rehabilitation assisting method according to the embodiment. First, the subject is equipped with the pressurizing unit 100 as shown in Fig. 41 (S300). Then gas is sent into the inside of the pressurizing unit 110, so that the expectoration part is squeezed (S302). At this time, the subject's respiratory state is measured simultaneously with the change of the atmospheric pressure of the pressurizing unit 110 inside (S304). Whether the pressure of pressurizing unit 110 inside is suitable is evaluated while the subject's respiratory state is observed, and the pressurization value may be changed if required (S306). The procedure is continued to N of S308 until the rehabilitation is completed, and the rehabilitation step is carried out. After the rehabilitation is completed (Y of S308), in order to measure a subject's respiratory state, the atmospheric pressure of pressurizing unit 110 inside is set at a suitable initial pressure (S310). Then, the respiratory function measurement is carried out (S312), and the effect of the rehabilitation is evaluated (S314).

Thus, the effect of the rehabilitation is evaluated since the rehabilitation assisting apparatus according to the embodiment also has the function as the measuring apparatus, and can measure the respiratory function during and after the rehabilitation. In the above-mentioned explanation, squeezing method was introduced as the physiotherapy, but rehabilitation and respiratory function measurement can be similarly performed when applying other method.

Although the present invention has been described with reference to embodiments, technical area of the present invention is not limited to the above described embodiments which are only exemplary. It is understood by those skilled in the art that there exist other various modifications to the combination of each component and process described above and that such modifications are encompassed by the scope of the present invention.

### INDUSTRIAL USABILITY

As has been described, the present invention can be utilized for a measuring system, a measuring apparatus and a measuring method for measuring respiratory function, an analyzing apparatus, analyzing server, analyzing program for analyzing the measurement result, and a rehabilitation assisting apparatus and a rehabilitation assisting method utilizing the measurement system.

## Claims

1. A measuring system (10) for measuring respiratory function **characterized in that** it includes:
a first measuring unit (100a) for detecting volume change of a first measurement part of a subject sequent to respiratory movement;
a second measuring unit (100b) for detecting volume change of a second measurement part of the subject sequent to respiratory movement;
a control unit (200) which acquires an output from said first measuring unit (100a) and said second measuring unit (100b); and
an analyzing unit (300) which analyzes the output which said control unit (200) acquires;
said first measuring unit (100a) and said second measuring unit (100b) respectively include:
a sensing unit (110) for sensing the volume change of the measurement part; and
a fixing unit (120) for arranging said sensing unit (110) to the measurement part;
wherein said fixing unit (120) can fix said sensing unit (110) in a manner of impressing said sensing unit (110) on the measurement part.

2. The measuring system (10) according to Claim 1
**characterized in that**:
the first measurement part is near thorax, and said first measuring unit (100a) detects the volume change near thorax sequent to costal respiration, and
the second measurement part is near abdomen, and said second measuring unit (100b) detects the volume change near abdomen sequent to abdominal respiration.

3. The measuring system (10) according to Claim 1 or 2 **characterized in that** said sensing unit (110) senses the volume change of the measurement part from change of pressure applied by said fixing unit (120) and the measurement part.

4. The measuring system (10) according to any one of Claims 1 to 3 **characterized in that**:
said sensing unit (110) has a bag-like form;
said fixing unit (120) has a belt-like form; and
said sensing unit (110) is impressed to the measurement part in such a manner that a body of the subject is wrapped around by said fixing unit (120).

5. The measuring system (10) according to Claim 3 or 4 **characterized in that**:
said sensing unit (110) has a cavity inside;
said measuring system (10) further includes a pressure sensor (230) for measuring air pressure in the cavity; and
the volume change of the measurement part is detected from change of the air pressure in the cavity.

6. The measuring system (10) according to Claim 5 **characterized in that** it further includes a pump (220) for sending gas into the cavity.

7. The measuring system (10) according to Claim 5 or 6 **characterized in that** it further includes an initial pressure adjusting unit (210) for adjusting the air pressure in the cavity to predetermined initial pressure by sending gas into the cavity before a measurement of respiratory function.

8. The measuring system (10) according to Claim 7 **characterized in that** said initial pressure adjusting unit (210) adjusts the initial pressure within a range where a ratio of the volume change to the change of the air pressure in the cavity is substantially constant.

9. The measuring system (10) according to Claim 7 **characterized in that** said initial pressure adjusting unit (210) adjusts the initial pressure within a range where a ratio of the volume change to the change of the air pressure in the cavity shifts substantially linear.

10. The measuring system (10) according to Claim 7
**characterized in that** said initial pressure adjusting unit (210) adjusts the initial pressure to be substantially constant when measuring a subject for a plurality of times.

11. The measuring system (10) according to Claim 7 or 10 **characterized in that** said initial pressure adjusting unit (210) adjusts the initial pressure to be substantially constant when measuring a plurality of subjects.

12. The measuring system (10) according to any one of Claims 1 to 11 **characterized in that** it further includes an indicating unit (260) which indicates suitable respiratory movement to a subject according to the kind of data which should be measured.

13. The measuring system (10) according to any one of Claims 1 to 11 **characterized in that** it further includes a condition input unit (290) which receives an input of information about a measurement condition.

14. The measuring system (10) according to Claim 13 **characterized in that** it further includes a measurement control unit (250) which controls said initial pressure adjusting unit (210) or said indicating unit (260) based on the measurement condition.

15. The measuring system (10) according to Claim 14 **characterized in that** said measurement control unit (250) determines the initial pressure which said initial pressure adjusting unit (210) adjusts based on the measurement condition.

16. The measuring system (10) according to Claim 14 or 15 **characterized in that** said measurement control unit (250) determines a content which said indicating unit (260) indicates to a subject based on the measurement condition.

17. The measuring system (10) according to any one of Claims 1 to 16 **characterized in that** it further includes a waveform generating unit (322) for generating respiratory waveform data which shows respiratory state of a subject from the volume change near thorax which the first measuring unit (100a) detects, and the volume change near diaphragm which the second measuring unit (100b) detects.

18. The measuring system (10) according to Claim 17 **characterized in that** said waveform generating unit (322) weights the volume change near thorax and the volume change near diaphragm with a predetermined ratio when generating the respiratory waveform data.

19. The measuring system (10) according to Claim 18 **characterized in that** it further includes a calculating unit (324,326,328,329) for calculating at least one respiratory function barometer among a lung capacity fraction, a forced expiratory curve, a forced lung capacity, a forced expiratory volume in one second, a forced expiratory rate in one second, a maximum mid-expiratory flow, a maximum ventilation volume, a flow volume curve, a peak expiratory flow rate, and a rate of abdomen contribution based on at least one of the volume change near thorax, the volume change near diaphragm and the respiratory waveform data.

20. The measuring system (10) according to Claim 19 **characterized in that** said calculating unit (324,326,328,329) calculates the respiratory function barometer, converting at least one of the volume change near thorax, the volume change near diaphragm, and the respiratory waveform data into respiratory volume.

21. The measuring system (10) according to any one of Claims 17 to 20 **characterized in that** it further includes a waveform characteristic extracting unit (330) which extracts a feature of waveform from at least one of the volume change near thorax, the volume change near diaphragm, and the respiratory waveform data.

22. The measuring system (10) according to any one of Claims 19 to 21 **characterized in that** it further includes a respiratory tract state judging unit (331) which judges state of constriction or blockage of a respiratory tract with reference to the respiratory waveform data, the forced expiration curve, or the flow volume curve.

23. The measuring system (10) according to Claim 22 **characterized in that** said respiratory tract state judging unit (331) judges that a respiratory tract of a subject is constricted or blocked in the case where the respiratory waveform data, the forced expiratory curve, or the flow volume curve has a waveform showing an increase of the air pressure in the cavity when the subject starts expiration.

24. The measuring system (10) according to Claim 23 **characterized in that** said respiratory tract state judging unit (331) judges a degree of constriction or blockage of the respiratory tract based on an aspect of increasing of the air pressure.

25. The measuring system (10) according to any one of Claims 1 to 24 **characterized in that** it further includes a database (350) which stores a medical view correspondent with at least one of a feature of the waveform of the respiratory waveform data, a feature of the waveform of the volume change near thorax, a feature of the waveform of the volume change near diaphragm, a difference of the waveform between the volume change near thorax and the volume change near diaphragm, a respiratory function barometer, and a personal data of a subject.

26. The measuring system (10) according to Claim 25 **characterized in that** it further includes a database referring unit (332) which acquires the medical view for the subject with reference to said database (350).

27. The measuring system (10) according to Claim 26 **characterized in that** it further includes a display unit (340) which displays the medical view.

28. A measuring apparatus (50) for measuring respiratory function **characterized in that** it includes:
a sensing unit (110) for sensing volume change of a measurement part of a subject sequent to respiratory movement; and
a fixing unit (120) for arranging said sensing unit (110) near the measurement part;
wherein said fixing unit (120) has a belt-like form and can fix said sensing unit (110) in a manner of impressing said sensing unit (110) on the measurement part; and
said sensing unit (110) senses the volume change of the measurement part from change of pressure applied by said fixing unit (120) and the measurement part.

29. A measuring apparatus (50) for measuring respiratory function **characterized in that** it includes:
a first measuring unit (100a) for detecting volume change of a first measurement part of a subject sequent to respiratory movement; and
a second measuring unit (100b) for detecting volume change of a second measurement part of the subject sequent to respiratory movement;
said first measuring unit (100a) and said second measuring unit (100b) respectively include:
a sensing unit (110) for sensing the volume change of the measurement part; and
a fixing unit (120) for arranging said sensing unit (110) near the measurement part;
wherein said fixing unit (120) has a belt-like form and can fix said sensing unit (110) in a manner of impressing said sensing unit (110) on the measurement part; and
said sensing unit (110) senses the volume change of the measurement part from change of pressure applied by said fixing unit (120) and the measurement part.

30. The measuring apparatus (50) according to Claim 28 or 29 **characterized in that** said sensing unit (110) has a cavity inside and includes a first connecting unit (112) for sending gas which is in the cavity to a pressure sensor (230) for measuring air pressure in the cavity.

31. The measuring apparatus (50) according to Claim 30 **characterized in that** said sensing unit (110) further includes a second connecting unit (114) for connecting with a pump (220) for sending gas into the cavity.

32. The measuring apparatus (50) according to Claim 31 **characterized in that** it further includes a control unit (200) which includes said pressure sensor (230) and said pump (220).

33. The measuring apparatus (50) according to Claim 32 **characterized in that** said control unit (200) further includes a recording unit (240) which records the air pressure in the cavity which said pressure sensor (230) measures.

34. The measuring apparatus (50) according to Claim 33 **characterized in that** said recording unit (240) records the air pressure in the cavity, or data converted from the air pressure into the volume or respiratory volume in an external recording medium.

35. The measuring apparatus (50) according to Claim 33 **characterized in that** said control unit (200) further includes a transfer unit (280) which transfers the air pressure in the cavity which said pressure sensor (230) measures to an analyzing apparatus (300) for analyzing respiratory function.

36. The measuring apparatus (50) according to any one of Claims 32 to 35 **characterized in that** it further includes an initial pressure adjusting unit (210) for adjusting the air pressure in the cavity to predetermined initial pressure by sending gas into the cavity before a measurement of respiratory function.

37. The measuring apparatus (50) according to Claim 36 **characterized in that** said initial pressure adjusting unit (210) adjusts the initial pressure within a range where a ratio of the volume change to the change of the air pressure in the cavity is substantially constant.

38. The measuring apparatus (50) according to Claim 36 **characterized in that** said initial pressure adjusting unit (210) adjusts the initial pressure within a range where a ratio of the volume change to the change of the air pressure in the cavity shifts substantially linear.

39. The measuring apparatus (50) according to Claim 36 **characterized in that** said initial pressure adjusting unit (210) adjusts the initial pressure to be substantially constant when measuring a subject for a plurality of times.

40. The measuring apparatus (50) according to Claim 36 or 39 **characterized in that** said initial pressure adjusting unit (210) adjusts the initial pressure to be substantially constant when measuring a plurality of subjects.

41. An analyzing apparatus (300) for analyzing respiratory function of a subject **characterized in that** it includes:
a measurement data acquiring unit (310) which acquires respiratory function measurement data of the subject; and
a calculating unit (324,326,328,329) for calculating at least one respiratory function barometer among a lung capacity fraction, a forced expiratory curve, a forced lung capacity, a forced expiratory volume in one second, a forced expiratory rate in one second, a maximum mid-expiratory flow, a maximum ventilation volume, a flow volume curve, a peak expiratory flow rate, and a rate of abdomen contribution.

42. The analyzing apparatus (300) according to Claim 41 **characterized in that**:
the respiratory function measurement data includes chest data acquired by measurement of volume change near thorax and abdomen data acquired by measurement of volume change near diaphragm; and
said analyzing apparatus (300) further includes a waveform generating unit (322) which generates respiratory waveform data which shows respiratory state of a subject from the chest data and the abdomen data.

43. The analyzing apparatus (300) according to Claim 42 **characterized in that** said waveform generating unit (322) weights the chest data near thorax and the abdomen data with a predetermined ratio when generating the respiratory waveform data.

44. The analyzing apparatus (300) according to Claim 42 or 43 **characterized in that** it further includes a waveform characteristic extracting unit (330) which extracts a feature of waveform from at least one of the volume change near thorax, the volume change near diaphragm, and the respiratory waveform data.

45. The analyzing apparatus (300) according to any one of Claims 41 to 44 **characterized in that** it further includes a respiratory tract state judging unit (331) which judges state of constriction or blockage of a respiratory tract with reference to the respiratory waveform data, the forced expiration curve, or the flow volume curve.

46. The analyzing apparatus (300) according to Claim 45 **characterized in that** said respiratory tract state judging unit (331) judges that a respiratory tract of a subject is constricted or blocked in the case where the respiratory waveform data, the forced expiratory curve, or the flow volume curve has a waveform showing an increase of the chest data or the abdomen data of the subject when the subject starts expiration.

47. The analyzing apparatus (300) according to Claim 46 **characterized in that** said respiratory tract state judging unit (331) judges a degree of constriction or blockage of the respiratory tract based on an aspect of increasing of the chest data or the abdomen data.

48. The analyzing apparatus (300) according to any one of Claims 44 to 47 **characterized in that** it further includes a database referring unit (332) which acquires a medical view for a subject with reference to a database (350) which stores the medical view correspondent with at least one of a feature of the waveform of the respiratory waveform data, a feature of the waveform of the volume change near thorax, a feature of the waveform of the volume change near diaphragm, a difference of the waveform between the volume change near thorax and the volume change near diaphragm, a respiratory function barometer, and a personal data of the subject.

49. A program **characterized in that** it makes a computer realize:
a function of acquiring respiratory function measurement data of a subject; and
a function of calculating at least one respiratory function barometer among a lung capacity fraction, a forced expiratory curve, a forced lung capacity, a forced expiratory volume in one second, a forced expiratory rate in one second, a maximum mid-expiratory flow, a maximum ventilation volume, a flow volume curve, a peak expiratory flow rate, and a rate of abdomen contribution.

50. The program according to Claim 49 **characterized in that**:
the respiratory function measurement data includes chest data acquired by measurement of volume change near thorax and abdomen data acquired by measurement of volume change near diaphragm; and
it further makes a computer realize a function of generating respiratory waveform data which shows respiratory state of a subject from the chest data and the abdomen data.

51. The program according to Claim 50 **characterized in that** it further makes a computer realize a function of weighting the chest data near thorax and the abdomen data with a predetermined ratio when generating the respiratory waveform data.

52. The program according to Claim 50 or 51 **characterized in that** it further makes a computer realize a function of extracting a feature of waveform from at least one of the volume change near thorax, the volume change near diaphragm, and the respiratory waveform data.

53. The program according to any one of Claims 49 to 52 **characterized in that** it further makes a computer realize a function of judging state of constriction or blockage of a respiratory tract with reference to the respiratory waveform data, the forced expiration curve, or the flow volume curve.

54. The program according to Claim 53 **characterized in that** it judges that a respiratory tract of a subject is constricted or blocked in the case where the respiratory waveform data, the forced expiratory curve, or the flow volume curve has a waveform showing an increase of the chest data or the abdomen data of the subject when the subject starts expiration.

55. The program according to Claim 54 **characterized in that** it judges a degree of constriction or blockage of the respiratory tract based on an aspect of increasing of the chest data or the abdomen data.

56. The program according to any one of Claims 52 to 55 **characterized in that** it further makes a computer realize a function of acquiring a medical view for a subject with reference to a database (350) which stores the medical view correspondent with at least one of a feature of the waveform of the respiratory waveform data, a feature of the waveform of the volume change near thorax, a feature of the waveform of the volume change near diaphragm, a difference of the waveform between the volume change near thorax and the volume change near diaphragm, a respiratory function barometer, and a personal data of the subject.

57. An analyzing server (400) for analyzing respiratory function of a subject **characterized in that** it comprises:
a database (350) stored respiratory function measurement data and a medical view correspondingly;
a receiving unit (430) which receives a requirement of referring said database (350) through a network (600);
a measurement data acquiring unit (310) which acquires the respiratory function measurement data of the subject;
a database referring unit (332) which acquires the medical view with reference to said database (350) based on the respiratory function measurement data; and
a transmitting unit (420) which transmits the medical view through the network (600).

58. The analyzing server (400) according to Claim 57 **characterized in that** the respiratory function measurement data includes chest data acquired by measurement of volume change near thorax and abdomen data acquired by measurement of volume change near diaphragm.

59. The analyzing server (400) according to Claim 58 **characterized in that** the respiratory function measurement data is respiratory waveform data generated using the chest data and the abdomen data.

60. The analyzing server (400) according to Claim 59 **characterized in that** the respiratory waveform data is generated by weighting the chest data and the abdomen data with a predetermined ratio.

61. A rehabilitation assisting apparatus (50) which assists rehabilitation of respiratory function **characterized in that** it comprises:
a pressurizing unit (100) which includes a pressurizing member (110) for squeezing a body of a subject; and a fixing unit (120) for arranging said pressurizing unit (100) to the body of the subject;
wherein said fixing unit (120) has a belt-like form and can fix said pressurizing member (110) in a manner of impressing said pressurizing member (110) on the body; and said pressurizing member (110) has a bag-like form and squeezes the body with increasing volume thereof by introducing gas therein.

62. The rehabilitation assisting apparatus (50) according to Claim 61 **characterized in that** said pressurizing member (110) is fixed so that said pressurizing member (110) squeezes an expectoration part of the subject.

63. The rehabilitation assisting apparatus (50) according to Claim 62 **characterized in that**:
said pressurizing unit (100) has a plurality of said pressurizing members (110); and
said rehabilitation assisting apparatus (50) further comprises a control unit (200) which controls volume of gas inside said pressurizing members (110) so that said pressurizing members (110) squeeze the expectoration part.

64. The rehabilitation assisting apparatus (50) according to any one of Claims 61 to 63 **characterized in that** it comprises a plurality of said pressurizing units (100); and said pressurizing units (110) is arranged at least near thorax and near diaphragm of the subject.

65. The rehabilitation assisting apparatus (50) according to any one of Claims 61 to 64 **characterized in that** it further comprises a pressure sensor (230) which measures air pressure inside said pressurizing member (110); and respiratory state of the subject is measured by sensing the volume change near thorax or diaphragm from change of the air pressure measured by said pressure sensor (230).

66. A method of measuring respiratory function **characterized in that** it includes:
a step of fixing a first measuring unit (100a) which includes a first sensing unit (110a) for sensing volume change of a first measurement part of a subject sequent to respiratory movement; and a first fixing unit (120) for arranging said first sensing unit (110a) to the first measurement part, in such a manner that said first fixing unit (120) pinches said first sensing unit (110a) between said first fixing unit (120) and the first measurement part;
a step of fixing a second measuring unit (100b) which includes a second sensing unit (110b) for sensing volume change of a second measurement part of the subject sequent to respiratory movement; and a second fixing unit (120) for arranging said second sensing unit (110b) to the second measurement part, in such a manner that said second fixing unit (120) pinches said second sensing unit (110b) between said second fixing unit (120) and the second measurement part;
a step of measuring simultaneously the volume change of the first measurement part sensed by said first sensing unit (110a) and the volume change of the second measurement part sensed by said second sensing unit (110b).

67. The method according to Claim 66 **characterized in that** the first measurement part is near thorax and the second measurement part is near diaphragm.

68. The method according to Claim 66 or 67 **characterized in that**:
said first fixing unit (120) and said second fixing unit (120) have belt-like forms;
said first sensing unit (110a) and said second sensing unit (110b) have bag-like forms;
said step of fixing fixes said first fixing unit (120) and said second fixing unit (120) in such a manner that a body of the subject is wrapped around by said first fixing unit (120) and said second fixing unit (120); and
said step of measuring measures the volume change by sensing air pressure inside said first sensing unit (110a) and said second sensing unit (110b).

69. The method according to Claim 68 **characterized in that**:
it further includes, between said step of fixing and said step of measuring, a step of adjusting the air pressure inside said sensing unit (110) to predetermined initial pressure by sending gas into said sensing unit (110); and
said step of measuring measures maintaining an amount of gas introduced into said sensing unit (110) in said adjusting.

70. A method of assisting rehabilitation of respiratory function **characterized in that** it includes:
a step of equipping a subject with a pressurizing unit (100) which comprises a pressurizing member (110) for squeezing a body of the subject and a fixing unit (120) for arranging the pressurizing member (110) to the body;
a step of squeezing the body of the subject by the pressurizing member (110);
a rehabilitation step of making the subject perform respiratory movement under squeezing;
a first measurement step of measuring respiratory state of the subject by sensing pressure applied to the pressurizing member (110) while said rehabilitation step.

71. The method according to Claim 70 **characterized in that**:
the fixing unit (120) has a belt-like form;
the pressurizing member (110) has a bag-like form;
said step of equipping fixes the pressurizing unit (100) in such a manner that the pressurizing member (110) is pinched between the fixing unit (120) and the body of the subject;
said step of squeezing squeezes the body with increasing volume of the pressurizing member (110) by introducing gas into the pressurizing member (110).

72. The method according to Claim 71 **characterized in that**:
said step of equipping equips the subject with the pressurizing unit (100) so that the pressurizing member (110) squeezes an expectoration part of the subject;
said step of squeezing squeezes the expectoration part by adjusting air pressure inside the pressurizing member (110).

73. The method according to any one of Claims 70 to 72 **characterized in that** it further includes after said rehabilitation step:
a step of adjusting air pressure inside the pressurizing member (110) to predetermined pressure;
a second measurement step of measuring respiratory state of the subject after rehabilitation by sensing the air pressure inside the pressurizing member (110).

74. The method according to any one of Claims 70 to 73 **characterized in that** it further includes a step of evaluating appropriate value of pressurizing to squeeze the body in said rehabilitation step based on the respiratory state of the subject measured in said first measurement step.

75. The method according to Claim 73 or 74 **characterized in that** it further includes a step of evaluating effect of said rehabilitation step based on the respiratory state of the subject measured in said second measurement step.
